(19) Europäisches Patentamt

European Patent Office

Office européen des brevets

(11) **EP 4 213 153 A1**

(12) # EUROPEAN PATENT APPLICATION
published in accordance with Art. 153(4) EPC

(43) Date of publication:
**19.07.2023 Bulletin 2023/29**

(21) Application number: **21888356.9**

(22) Date of filing: **09.10.2021**

(51) International Patent Classification (IPC):
**G16C 20/50** (2019.01)

(52) Cooperative Patent Classification (CPC):
**G16C 20/10; G16C 20/70**

(86) International application number:
**PCT/CN2021/122724**

(87) International publication number:
**WO 2022/095659 (12.05.2022 Gazette 2022/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.11.2020 CN 202011218991**

(71) Applicant: **Tencent Technology (Shenzhen)
Company Limited
Shenzhen, Guangdong, 518057 (CN)**

(72) Inventors:
• **FU, Yue**
  **Shenzhen, Guangdong 518057 (CN)**
• **HSIEH, Chang-Yu**
  **Shenzhen, Guangdong 518057 (CN)**
• **LIAO, Benben**
  **Shenzhen, Guangdong 518057 (CN)**
• **HAO, Jianye**
  **Shenzhen, Guangdong 518057 (CN)**
• **ZHANG, Sheng Yu**
  **Shenzhen, Guangdong 518057 (CN)**

(74) Representative: **Eisenführ Speiser
Patentanwälte Rechtsanwälte PartGmbB
Postfach 31 02 60
80102 München (DE)**

(54) **METHOD AND APPARATUS FOR TRAINING NEURAL NETWORK FOR DETERMINING MOLECULE RETROSYNTHESIS ROUTE**

(57) A method for training a neural network for determining a molecule retrosynthesis route, comprises: for a plurality of molecules, when determining retrosynthesis routes of the molecules, using a hierarchical learning concept, dividing a training process, which needs a relatively deep search of a molecule retrosynthesis route, into multiple layers for training, replacing a complete retrosynthesis reaction process with the multiple layers of molecule retrosynthesis routes, and upon the completion of the training of one layer of molecule retrosynthesis route, selecting, by means of a molecular filtering method, representative molecules as starting molecules of the next layer of molecule retrosynthesis route. The present invention can effectively improve the efficiency of searching for a molecule retrosynthesis route, such that accurate molecule cost value information can be extracted more efficiently. In a hierarchical manner, a large amount of calculation overheads caused by determining a molecule retrosynthesis route are greatly reduced, and on the basis of ensuring the accuracy of the molecule retrosynthesis route, the time for determining the molecule retrosynthesis route is reduced.

**(Cont. next page)**

EP 4 213 153 A1

The server determines a first decomposition path of each of a plurality of first molecules based on molecular expression information of each of the plurality of first molecules — 301

The server obtains a first cost dictionary based on the first decomposition paths of the first molecules, the first cost dictionary comprising the molecular expression information of each of the first molecules and cost value information of each of the first molecules, and the cost value information of each first molecule representing a cost value required to decompose the first molecule according to the first decomposition path of the first molecule — 302

The server determines molecular expression information of at least one second molecule based on the first decomposition paths of the first molecules, each of the second molecules being a molecule that is capable of being decomposed into obtainable molecules — 303

The server determines a plurality of third molecules from the second molecules, each of the third molecules representing a class of the second molecules — 304

The server obtains a second cost dictionary based on a second decomposition path of each of the third molecules, the second cost dictionary comprising molecular expression information of each of the third molecules and cost value information of each of the third molecules, and the cost value information of each third molecule representing a cost value required to decompose the third molecule according to the second decomposition path of the third molecule — 305

The server performs training based on the first cost dictionary and the second cost dictionary to obtain a target neural network, the target neural network being configured to output cost value information of a target molecule according to input molecular expression information of the target molecule — 306

FIG. 3

**Description**

RELATED APPLICATION

This application claims priority to Chinese Patent Application No. 2020112189912, entitled "METHOD AND APPARATUS FOR TRAINING NEURAL NETWORK FOR DETERMINING MOLECULE RETROSYNTHESIS ROUTE" filed with the China National Intellectual Property Administration on November 4, 2020, which is incorporated herein by reference in its entirety.

FIELD OF THE TECHNOLOGY

This application relates to the technical field of artificial intelligence, and in particular, to a training method and apparatus for a neural network for determining a molecular retrosynthetic route, and equipment and a readable storage medium.

BACKGROUND OF THE DISCLOSURE

In recent years, with the rapid development of artificial intelligence technology, it has been gradually introduced into and plays an important role in various scientific areas. In the field of chemistry, since chemical reactions are endlessly variable under different conditions, a reasonable organic synthetic route is needed to be designed by researchers who consumes a lot of time and efforts when compound molecules are prepared in the past. Where organic synthetic routes are designed by researchers with the aid of artificial intelligence technology, efficiency in developing chemical drug molecules and other compounds by researchers can be greatly improved.

The current methods for designing molecular retrosynthetic routes based on artificial intelligence include the following: One method includes a random search step based on the Monte Carlo Tree Search (MCTS) algorithm until a solution is found or a maximum depth is reached, and symbolic artificial intelligence is introduced to complete the design of the molecular retrosynthetic route. Another method is to determine a template selection strategy for each step of the molecular retrosynthetic reaction based on deep reinforcement learning technology, and finally obtain a molecular retrosynthetic route. Another method is to use a distributed training architecture in combination with deep reinforcement learning technology to accelerate the construction of an optimal molecular retrosynthetic route and the fitting of a network of a cost value function, and implement the design of a training set molecular retrosynthetic route through the network.

However, it takes a long time to design the molecular retrosynthetic route using the above methods, and the above methods requires determining a maximum exploration height in the early stage of building a molecular retrosynthetic tree. Consequently, if the maximum exploration height is too small, it is difficult to complete the construction of molecular retrosynthetic trees within a limited height for some complex molecules. On the contrary, if the maximum exploration height is too large, the time required will increase exponentially, resulting in low efficiency and low accuracy of molecular retrosynthetic route design.

SUMMARY

Embodiments of this application provide a training method and apparatus for a neural network for determining a molecular retrosynthetic route, a method for determining a molecular retrosynthetic route, an apparatus, a device, and a readable storage medium.

One aspect provides a training method for a neural network for determining a molecular retrosynthetic route, performed by a computer device, the method including:

determining a first decomposition path of each of a plurality of first molecules based on molecular expression information of each of the plurality of first molecules;

obtaining a first cost dictionary based on the first decomposition paths of the first molecules, the first cost dictionary comprising the molecular expression information of each of the first molecules and cost value information of each of the first molecules, and the cost value information of each first molecule representing a cost value required to decompose the first molecule according to the first decomposition path of the first molecule;

determining molecular expression information of at least one second molecule based on the first decomposition paths of the first molecules, each of the second molecules being a molecule that is capable of being decomposed into obtainable molecules, among molecules obtained by decomposing each of the first molecules based on the first decomposition path of each of the first molecules;

determining a plurality of third molecules from the second molecules, each of the third molecules representing a class of the second molecules;

obtaining a second cost dictionary based on a second decomposition path of each of the third molecules, the second cost dictionary comprising molecular expression information of each of the third molecules and cost value information of each of the third molecules, and the cost value information of each third molecule representing a cost value required to decompose the third molecule according to the second decomposition path of the third molecule; and

performing training based on the first cost dictionary and the second cost dictionary to obtain a target neural network, the target neural network being configured to output cost value information of a target molecule according to input molecular expression information of the target molecule, the cost value information of the target molecule being used for synthesizing a retrosynthetic route for the target molecule.

Another aspect provides a method for determining a molecular retrosynthetic route, performed by a computer device, the method including:

receiving molecular expression information of a target molecule, the molecular expression information representing a three-dimensional chemical structure of the target molecule;

inputting the molecular expression information of the target molecule into a neural network, wherein the neural network is for determining a molecular retrosynthetic route;

determining a target decomposition path of the target molecule based on the neural network for determining a molecular retrosynthetic route, the target decomposition path being a decomposition path with a minimum decomposition cost value among at least one decomposition path of the target molecule; and

obtaining molecular retrosynthetic route information of the target molecule based on the target decomposition path.

Another aspect provides a training apparatus for a neural network for determining a molecular retrosynthetic route, disposed in a computer device, the apparatus including:

a first determining module, configured to determine a first decomposition path of each of a plurality of first molecules based on molecular expression information of each of the plurality of first molecules;

a first cost dictionary generation module, configured to obtain a first cost dictionary based on the first decomposition paths of the first molecules, the first cost dictionary comprising the molecular expression information of each of the first molecules and cost value information of each of the first molecules, and the cost value information of each first molecule representing a cost value required to decompose the first molecule according to the first decomposition path of the first molecule;

a second determining module, configured to determine molecular expression information of at least one second molecule based on the first decomposition paths of the first molecules, each of the second molecules being a molecule that is capable of being decomposed into obtainable molecules, among molecules obtained by decomposing each of the first molecules based on the first decomposition path of each of the first molecules;

a third determining module, configured to determine a plurality of third molecules from the second molecules, each of the third molecules representing a class of the second molecules;

a second cost dictionary generation module, configured to obtain a second cost dictionary based on a second decomposition path of each of the third molecules, the second cost dictionary comprising molecular expression information of each of the third molecules and cost value information of each of the third molecules, and the cost value information of each third molecule representing a cost value required to decompose the third molecule according to the second decomposition path of the third molecule; and

a training module, configured to perform training based on the first cost dictionary and the second cost dictionary to obtain a target neural network, the target neural network being configured to output cost value information of a target molecule according to input molecular expression information of the target molecule, the cost value information of the target molecule being used for synthesizing a retrosynthetic route for the target molecule.

Another aspect provides an apparatus for determining a molecular retrosynthetic route, disposed in a computer device, the apparatus including:

a receiving module, configured to receive molecular expression information of a target molecule, the molecular expression information representing a three-dimensional chemical structure of the target molecule;

an input module, configured to input the molecular expression information of the target molecule into a neural network, wherein the neural network is for determining a molecular retrosynthetic route;

a decomposition path determining module, configured to determine a target decomposition path of the target molecule based on the neural network for determining a molecular retrosynthetic route, the target decomposition path being a decomposition path with a minimum decomposition cost value among at least one decomposition path of the target molecule; and

a route determining module, configured to obtain molecular retrosynthetic route information of the target molecule based on the target decomposition path.

Another aspect provides a computer device, including one or more processors and a memory, the memory being configured to store at least one computer-readable instruction, the at least one computer-readable instruction being loaded and executed by the one or more processors to implement the operations performed in the methods according to the embodiments of this application.
Another aspect provides one or more computer-readable storage media, storing at least one computer-readable instruction, the at least one computer-readable instruction being loaded and executed by one or more processors to implement the operations performed in the methods according to the embodiments of this application.
Another aspect provides a computer program product, including computer instructions, the computer instructions being stored in a computer-readable storage medium. One or more processors of the computer device reads the computer-readable instructions from the computer-readable storage medium, and the one or more processor execute the computer-readable instructions to cause the computer device to perform the steps in the methods according to the above optional implementations.
Details of one or more embodiments of this application are provided in the accompanying drawings and descriptions below. Based on the specification, the accompanying drawings, and the claims of this application, other features, objectives, and advantages of this application become clearer.

BRIEF DESCRIPTION OF THE DRAWINGS

To describe technical solutions in embodiments of this application more clearly, the following briefly introduces accompanying drawings describing the embodiments. Apparently, the accompanying drawings in the following description show merely some embodiments of this application, and a person of ordinary skill in the art may still derive other accompanying drawings according to the accompanying drawings without creative efforts.

FIG. 1 is a schematic diagram of an implementation environment of a training method for a neural network for determining a molecular retrosynthetic route according to an embodiment of this application.

FIG. 2 is an architecture diagram of building a molecular retrosynthetic tree based on a hierarchical manner according to an embodiment of this application.

FIG. 3 is a flowchart of a training method for a neural network for determining a molecular retrosynthetic route according to an embodiment of this application.

FIG. 4 is a flowchart of another training method for a neural network for determining a molecular retrosynthetic route according to an embodiment of this application.

FIG. 5 is a flowchart of a method for obtaining a second cost vocabulary according to an embodiment of this application.

FIG. 6 is an architecture diagram of a training method for a neural network for determining a molecular retrosynthetic route according to an embodiment of this application.

FIG. 7 is a flowchart of a method for determining a molecular retrosynthetic route according to an embodiment of this application.

FIG. 8 is a block diagram of a training apparatus for a neural network for determining a molecular retrosynthetic route according to an embodiment of this application.

FIG. 9 is a schematic structural diagram of a server according to an embodiment of this application.

DESCRIPTION OF EMBODIMENTS

To make objectives, technical solutions, and advantages of this application clearer, the following further describes implementations of this application in detail with reference to the accompanying drawings. Exemplary embodiments are described in detail herein, and examples thereof are shown in the accompanying drawings. When the following descriptions are made with reference to the accompanying drawings, unless otherwise indicated, the same numbers in different accompanying drawings represent the same or similar elements. The following implementations described in the following exemplary embodiments do not represent all implementations that are consistent with this application. Instead, they are merely examples of apparatuses and methods consistent with aspects related to this application as recited in the appended claims. The following briefly introduces technologies and terms that may be used in the embodiments of this application.

Artificial Intelligence (AI) is a theory, method, technology, and application system that uses a digital computer or a machine controlled by the digital computer to simulate, extend, and expand human intelligence, perceive an environment, acquire knowledge, and use knowledge to obtain an optimal result. In other words, AI is a comprehensive technology in computer science. This technology attempts to understand the essence of intelligence and produce a new intelligent machine that can react in a manner similar to human intelligence. AI is to study the design principles and implementation methods of various intelligent machines, so that the machines can perceive, infer, and make decisions.

ML(Machine Learning) is a multi-field interdiscipline and relates to a plurality of disciplines such as probability theory, statistics, approximation theory, convex analysis, and algorithm complexity theory. ML specializes in studying how a computer simulates or implements a human learning behavior to acquire new knowledge or skills, and reorganize an existing knowledge structure, so as to keep improving its performance. ML is the core of AI, is a basic way to make a computer intelligent, and is applied to various fields of AI. ML and deep learning generally include technologies such as an artificial neural network, a belief network, reinforcement learning, transfer learning, inductive learning, and learning from demonstrations.

A Markov decision process (MDP) is a mathematical model of sequential decision and is used for simulating a random strategy and returns that the intelligent agent can implement in an environment in which a system state has a Markov property. The MDP is constructed based on a set of interactive objects, that is, the intelligent agent and the environment, including elements such as states, actions, strategies, and rewards. In simulation of the MDP, the intelligent agent perceives the current system state and implements actions on the environment according to the strategy, thereby changing the state of the environment and obtaining awards. The accumulation of awards over time is referred to as rewards.

The molecular expression information is information for representing a three-dimensional chemical structure of a molecule. For example, the molecular expression information is a simplified molecular input line entry specification (SMILES) of the molecule, that is, the chemical structure of the molecule is represented by a character string. For another example, the molecular expression information is a molecular graph used for representing the molecular structure, as shown in Table 1. Table 1 shows the properties of nodes and edges commonly used in the molecular graph.

Table 1

| Category | Property | Description |
|---|---|---|
| Node | Atom type | Carbon, nitrogen, oxygen, hydrogen, fluorine, sulfur, chlorine, etc. (one-hot encoding) |
| | Atomic number | Number of protons (integer) |
| | Positive valence | Receive electron (binary) |
| | Negative valence | Contributed electron (binary) |
| | Aromatic | In aromatic (binary) |
| | Hybridization | sp, sp2, sp3 (one-hot encoding or zero) |
| | Number of hydrogen atoms | (integer) |
| Edge | Chemical bond type | Single bond, double bond, triple bond, aromatic (one-hot encoding) |
| | Atomic distance | (real number) |

A Jaccard similarity coefficient is used to compare the similarities and differences between finite sample sets. A larger value of the Jaccard similarity coefficient indicates a higher sample similarity. A Tanimoto coefficient is extended from the Jaccard coefficient, and is also known as a generalized Jaccard similarity coefficient.

A Synthetic Accessibility score (SA Score) is a method for quickly assessing the difficulty in synthesis of a large number of compounds based on the complexity of the molecules. In this method, based on the assumption that "substructures that frequently appear are easy to synthesize", frequencies of extended-connectivity fingerprints of diameter 4 are weighted among 1 million compounds obtained from a bioactivity database of small organic molecules (PubChem), and the frequency of occurrence and molecular complexity are used as evaluation indicators to calculate the synthetic accessibility of the molecule. The value of the synthetic accessibility score is standardized as 1 (easy) to 10 (hard). In the application the SA Score is also used as a decomposition accessibility score for assessing the ease of decomposition of a large number of compounds. For a same molecule or compound, the decomposition accessibility score is the same as the SA score.

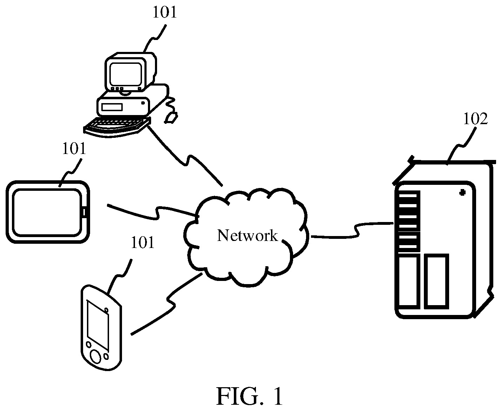

The following describes an implementation environment of the training method provided in the embodiments of this application. FIG. 1 is a schematic diagram of an implementation environment of a training method for a neural network for determining a molecular retrosynthetic route according to an embodiment of this application. The implementation environment includes: a terminal device 101 and a server 102.

The terminal 101 and the server 102 can be directly or indirectly connected in a wired or wireless communication manner, which is not limited in this application. In one embodiment, the terminal 101 is a smartphone, a tablet computer, a laptop computer, a desktop computer, or the like, but is not limited thereto. The terminal 101 can provide the server 102 with basic information required by the method for determining a molecular retrosynthetic route, such as molecular expression information (including a molecular graph structure, simplified molecular input line entry specification, etc.), molecular cost value reference information (such as molecular synthetic accessibility score) and molecular retrosynthesis reference information (such as molecular decomposition scheme), etc.

The server 102 can be an independent physical server, or can be a server cluster including a plurality of physical servers or a distributed system, or can be a cloud server providing basic cloud computing services, such as a cloud service, a cloud database, cloud computing, a cloud function, cloud storage, a network service, cloud communication, a middleware service, a domain name service, a security service, a content delivery network (CDN), big data, and an artificial intelligence platform. The server 102 is configured to execute the method for determining a molecular retrosynthetic route provided in the embodiments of this application, and to perform neural network training based on the basic information provided by the terminal 101. In one embodiment, the server 102 is capable of hosting a Linux operating system and GPU computing resources.

In one embodiment, in the process of training the neural network for determining a molecular retrosynthetic route, the server 102 is responsible for the main computing work, and the terminal 101 is responsible for the secondary computing work. Alternatively, the server 102 is responsible for secondary computing work, and the terminal 101 is responsible for primary computing work. or, the server 102 or the terminal 101 can be responsible for the computing work alone.

In one embodiment, the above training process adopts distributed training, for example, multiple computing nodes are respectively used for training. The server 102 includes a training server. The training server is a server cluster, including a plurality of servers serving as computing nodes. Each computing node performs a part of a training task respectively. A neural network model obtained through training can be transmitted to a target server to provide users with corresponding functions.

In one embodiment, the terminal 101 generally refers to one of a plurality of terminals. In this embodiment, the terminal 101 is merely used as an example for description. It can be understood by a person skilled in the art that there may be more terminals 101 and servers 1021. For example, there may be dozens of or hundreds of or more terminals 101. In this case, the implementation environment of the method for determining a molecular retrosynthetic route may further include other terminals. The quantity and the device type of the terminals are not limited in the embodiments of this application.

In an embodiment, a standard communication technology and/or protocol is used for the foregoing wireless network or the wired network. The network is usually the Internet, but can alternatively be any other networks, including but not limited to a local area network (LAN), a metropolitan area network (MAN), a wide area network (WAN), a mobile, wired, or wireless network, or any combination of a dedicated network or a virtual dedicated network). In some embodiments, technologies and/or formats, such as hypertext markup language (HTML) and extensible markup language (XML), are used for representing data exchanged through a network. In addition, all or some links can be encrypted by using conventional encryption technologies such as secure socket layer (SSL), transport layer security (TLS), virtual private network (VPN), and Internet Protocol security (IPsec). In other embodiments, custom and/or dedicated data communication technologies can also be used in place of or in addition to the foregoing data communication technologies.

In the embodiments of this application, a method for determining a molecular retrosynthetic route is provided, which adopts hierarchical reinforcement learning. In the chemical field, retrosynthesis analysis is an important method for resolving a molecular synthetic route, and is also the simplest and most basic method for designing a molecular synthetic route. The essence lies in the decomposition of a target molecule. The structure of the target molecule is analyzed and gradually decomposed into simpler and easier-to-synthesize intermediates and raw materials, thereby completing the design of a molecular synthetic route. The intermediates refer to precursor compounds required for the synthesis of the target molecule, that is, organic compounds that are not readily available in the market and need to be synthesized. The raw materials refer to relatively simple organic compounds that are readily available in the market for synthesizing the target molecule. The exploration of a retrosynthetic route of a molecule is the process of constructing a retrosynthetic tree for the molecule. In the related art, training for the construction of a retrosynthetic tree of an existing molecule is generally performed based on a preset maximum exploration height. This method leads to the fact that if the maximum exploration height is too small, some more complex molecules are very difficult to perform. Consequently, if the maximum exploration height is too small, some relatively it is difficult to complete the construction of molecular retrosynthetic trees within a limited height for some for complex molecules. On the contrary, if the maximum exploration height is too large, the time required for construction will increase exponentially. In the training process for the construction of a molecular retrosynthetic tree in the embodiments of this application, hierarchical construction is adopted, which can greatly reduce the large amount of calculation and time overheads required in the process of constructing a molecular retrosynthetic tree. For a detailed description, reference may be made to the following embodiments.

The following embodiments are all described using an example where the process of constructing a molecular retrosynthetic route is divided into two layers. However, the embodiments of this application are not limited thereto, but the process of constructing a molecular retrosynthetic tree may be divided into multiple layers, which is not limited in the embodiments of this application.

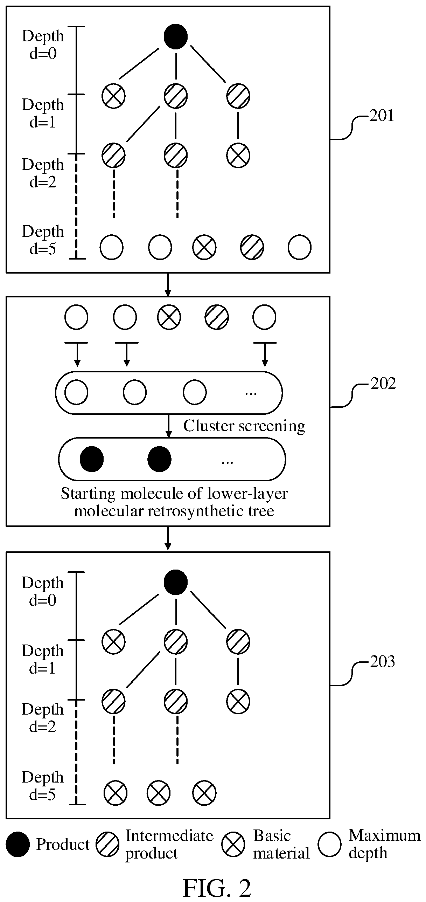

For example, referring to FIG. 2, FIG. 2 is an architecture diagram of building a molecular retrosynthetic tree based on a hierarchical manner according to an embodiment of this application. As shown in FIG. 2, taking a maximum depth of the molecular retrosynthetic tree being 10 as an example, the construction of the entire molecular retrosynthetic tree is divided into upper layer and lower layer, and two smaller molecular retrosynthetic trees are used to replace the complete retrosynthetic reaction process. After the construction of the upper-layer molecular retrosynthetic tree is completed, a representative molecule is selected by molecular clustering and screening and used as a starting molecule in the lower-layer molecular retrosynthetic tree, which effectively improves the exploration efficiency of the molecular retrosynthetic route, whereby accurate molecular cost information is more efficiently extracted.

A method for determining a molecular retrosynthetic route according to an embodiment of this application is described in detail below.

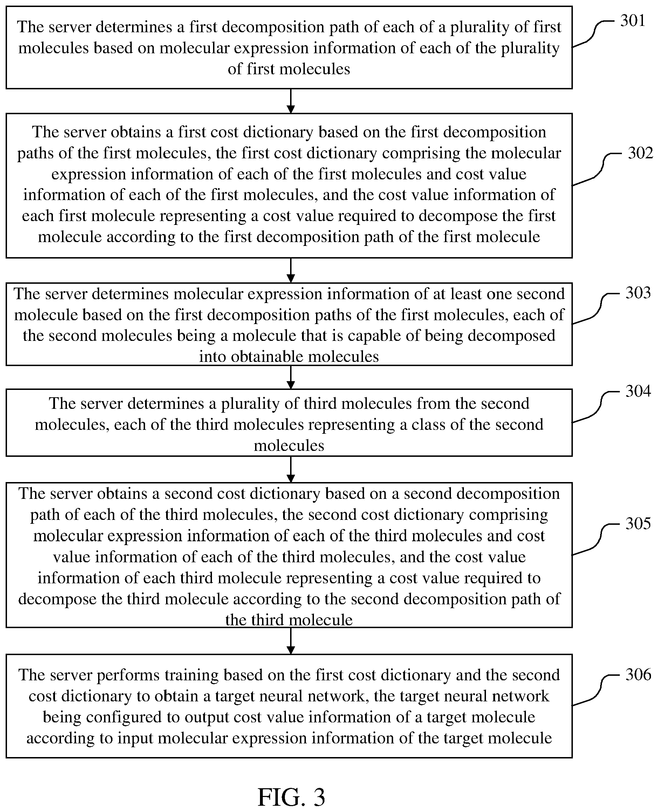

FIG. 3 is a flowchart of a method for determining a molecular retrosynthetic route according to an embodiment of this application. As shown in FIG. 3, this embodiment of this application is described using an example where the method is applied to a server. The method includes the following steps:

In step 301, the server determines a first decomposition path of each of a plurality of first molecules based on molecular expression information of each of the plurality of first molecules, a path depth of the first decomposition path being less than or equal to a target depth.

In an embodiment of this application, the first molecules are existing molecules. The molecular expression information represents a three-dimensional chemical structure of a molecule. For example, the molecular expression information is a simplified molecular input line entry specification (SMILES) of the molecule, that is, the chemical structure of the molecule is represented by a character string. For another example, the molecular expression information is a molecular map representing the molecular structure, which is not limited in the embodiments of this application. The first decomposition path is a path that requires the least cost value to decompose the first molecule until the target decomposition condition is met.

In an embodiment, the target decomposition condition is that a path depth of a decomposition path, of each first molecule, obtained in at least one decomposition level is less than a target depth and there is no method for decomposing molecules obtained from the decomposition path, or is that the path depth of the decomposition path of each first molecule obtained in at least one decomposition level is equal to the target depth. The target depth is a depth threshold preset by the server. For example, the depth threshold is set to 5.

In an embodiment, the first decomposition path of each of the plurality of first molecules is determined based on molecular expression information of each of the plurality of first molecules by using a first cost value function. The input of the first cost value function is molecular expression information of the first molecule, and the output of the first cost value function is a decomposition cost value required to decompose the first molecule according to the first decomposition path. The decomposition cost value, or somewhere referred to as cost value or cost, represents difficulty in decomposition of a molecule. For example, the decomposition cost value ranges from 0 to 100 or 0 to 50, where the larger the value, the more difficult it is, or the smaller the value, the more difficult it is. In an embodiment, the decomposition cost value or the cost value is the Synthetic Accessibility score.

The first cost value function is initialized by using cost value reference information. The cost value reference information represents difficulty in decomposition of a molecule. The cost value reference information has a same value range and a same value meaning as the decomposition cost value. For example, the decomposition cost value is the Synthetic Accessibility score, and the cost value reference information is also the Synthetic Accessibility score.

In step 302, the server obtains a first cost dictionary based on the first decomposition paths of the first molecules, the first cost dictionary includes the molecular expression information of each of the first molecules and cost value information of each of the first molecules, and the cost value information of each first molecule represents a cost value required to decompose the first molecule according to the first decomposition path of the first molecule.

In the embodiments of this application, the molecular expression information and the corresponding cost value information in the first cost dictionary exist in a one-to-one correspondence manner.

In step 303, the server determines molecular expression information of at least one second molecule based on the first decomposition paths of the first molecules, each of the second molecules being a molecule that can be decomposed into obtainable molecules.

In the embodiments of this application, after each first molecule is decomposed based on the corresponding first decomposition path, multiple molecules can be obtained. Among the multiple molecules, some molecules can be further decomposed, and there is a decomposition path. These molecules are determined as second molecules. That is, each of the second molecules is a molecule that can be decomposed into obtainable molecules among molecules obtained by decomposing the corresponding first molecule based on the first decomposition path.

In step 304, the server determines a plurality of third molecules from the second molecules, each of the third molecules represents a class of the second molecules.

In the embodiments of this application, the second molecules are divided into multiple sets based on structural similarities between molecules. The second molecules in each set have similar molecular structures. A third molecule is determined from each set. The third molecule is a representative molecule in the set to which the third molecule belongs.

In step 305, the server obtains a second cost dictionary based on second decomposition paths of the third molecules, the second cost dictionary includes the molecular expression information of each of the third molecules and cost value information of each of the third molecules, and the cost value information of each third molecule representing a cost value required to decompose the third molecule according to the second decomposition path of the third molecule.

In the embodiments of this application, the second decomposition path is a path that requires the least cost value to decompose the third molecule until the target decomposition condition is met. The molecular expression information and the corresponding cost value information in the second cost dictionary exist in a one-to-one correspondence manner. The method for obtaining the second decomposition path is the same as the method for obtaining the first decomposition path, which is not repeated here.

In step 306, the server performs training based on the first cost dictionary and the second cost dictionary to obtain a target neural network, the target neural network being configured to output cost value information corresponding to a target molecule according to input molecular expression information of the target molecule.

It can be understood that the decomposition path corresponding to the cost value information corresponding to the target molecule is the decomposition path with the lowest decomposition cost value among all possible decomposition paths of the target molecule. Therefore, a retrosynthetic route for the target molecule may be synthesized based on the cost value information corresponding to the target molecule.

In one embodiment, the target molecule may be decomposed based on the decomposition path corresponding to the cost value information, and molecular retrosynthetic route information may be obtained according to a result of the decomposition.

In the embodiments of this application, the server performs training based on the first cost dictionary to obtain a first neural network, performs training based on the second cost dictionary to obtain a second neural network, and finally combines the two neural networks to obtain the target neural network.

In the embodiments of this application, through steps 301 to 306, after dividing the exploration of the retrosynthetic routes of multiple molecules into multiple layers, the server respectively obtains a cost dictionary corresponding to each layer according to an Lth layer and an (L+1)th layer obtained, where L is greater than or equal to 1. For each layer obtained in the exploration process, a corresponding cost dictionary can be obtained. Correspondingly, the server trains the neural network based on the first cost dictionary, the second cost dictionary and the cost dictionaries obtained by the first L-1 layers to obtain a trained neural network, so as to obtain multiple layers of molecular retrosynthetic routes of the molecule. In the embodiments of this application, a training method for a neural network for determining a molecular retrosynthetic route is provided. When a retrosynthetic route of each of a plurality of molecules is determined, a concept of hierarchical learning is adopted. A training process of a molecular retrosynthetic route requiring deeper exploration is split into multiple layers for training to accelerate the training, and the complete retrosynthetic reaction process is replaced by multiple layers of molecular retrosynthetic routes. After the training of one layer of molecular retrosynthesis route is completed, a representative molecule is selected by molecular screening and used as a starting molecule in a next layer of molecular retrosynthetic route, which effectively improves the exploration efficiency of the molecular retrosynthetic route, whereby accurate molecular cost information is more efficiently extracted. The layered approach greatly reduces the computational overhead brought about by determining the molecular retrosynthetic route, and reduces the time for determining the molecular retrosynthetic route while the accuracy of the molecular retrosynthetic route is ensured.

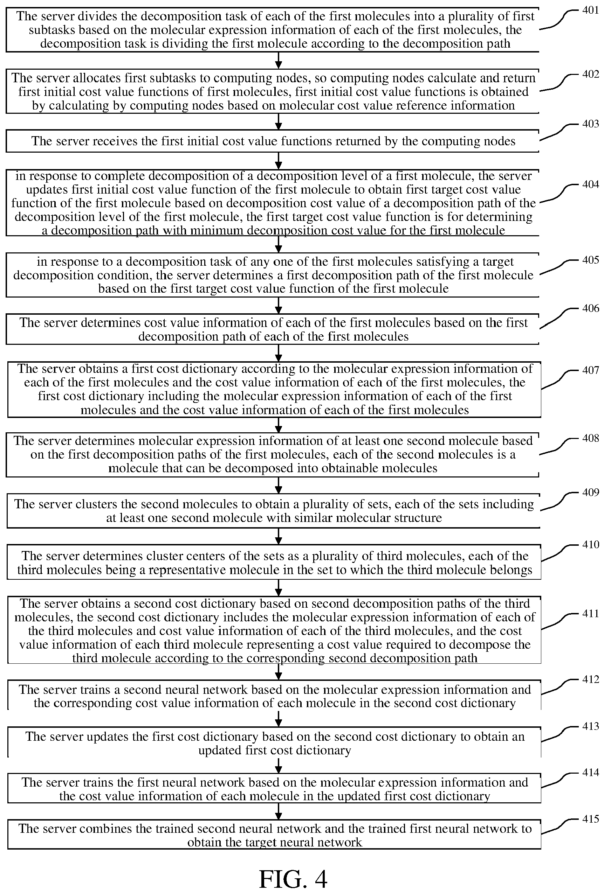

FIG. 4 is a flowchart of another training method for a neural network for determining a molecular retrosynthetic route according to an embodiment of this application. As shown in FIG. 4, this embodiment of this application is described using an example where the method is applied to a server and a molecular retrosynthetic route is divided into two layers. The method includes the following steps:

In step 401, the server divides the decomposition task of each of the first molecules into a plurality of first subtasks based on the molecular expression information of each of the first molecules, the decomposition task is dividing the first molecule according to the decomposition path.

In the embodiments of this application, the decomposition task is to gradually decompose a molecule into at least one simpler and easier-to-synthesize molecule according to the decomposition path through the analysis of the molecular structure of the molecule based on molecular expression information of the molecule.

In an optional implementation, the server is associated with a molecule database, where the molecule database is used to store molecular expression information of existing molecules. The server can extract the molecular expression information of each first molecule from the molecule database, generate a decomposition task of each first molecule based on the molecular expression information of each first molecule, and then divide the decomposition task of each first molecule into multiple first subtasks. Each first subtask includes the decomposition task of at least one first molecule.

On the premise that one first molecule corresponds to one decomposition task, the division of the decomposition tasks of the multiple first molecules by the server includes any one of the following implementations.

In a first optional implementation, the server performs an average division according to a current quantity of first molecules and a current quantity of computing nodes. For example, the current quantity of first molecules is divided by the current quantity of computing nodes, and the obtained value is used to determine the quantity of first subtasks in each computing node. Each first subtask includes the same quantity of decomposition tasks of first molecules.

In a second optional implementation, the server divides the decomposition tasks by levels of complexity of chemical three-dimensional structures of molecules according to the molecular expression information of the first molecules, for example, allocates a decomposition task of the first molecule with a single benzene ring into a first subtask, and allocates a decomposition task of the first molecule with more than one benzene ring into another first subtask, and so on. Levels of complexity of chemical three-dimensional structures of the first molecules corresponding to one first subtask are similar.

In a third optional implementation, the server divides the decomposition tasks according to the current quantity of computing nodes and computing capabilities of the computing nodes. For example, when a quantity of subtasks currently running on a computing node is greater than a threshold, the decomposition tasks of 10 first molecule are allocated to one first subtask; when the quantity of subtasks currently running on a computing node is less than the threshold, the decomposition tasks of 100 first molecules are allocated to another first subtask; and so on. Each first subtask includes a different quantity of decomposition tasks of first molecules.

The manner of dividing the decomposition tasks of the plurality of first molecules is not specifically limited in the embodiments of this application. Any of the optional methods provided above may be adopted, or any of the optional methods may be combined to obtain a more complex division manner.

In step 402, the server allocates the first subtasks to a plurality of computing nodes, so that the computing nodes calculate and return the first initial cost value functions of the first molecules, the first initial cost value functions is obtained by calculating by the corresponding computing nodes based on molecular cost value reference information.

In the embodiments of this application, the server can process data based on multiple computing nodes at the same time. The server assigns multiple first subtasks to multiple computing nodes, with one computing node being responsible for the decomposition task of at least one first molecule. The parallel computing mode using multiple computing nodes can accelerate the training process and improve the training speed. The first initial cost value function is an initial cost value function of each first molecule calculated by the computing node based on the molecular expression information of each first molecule and by using the molecular cost value reference information. The molecular cost value reference information is used for representing synthetic accessibility of the molecule. The cost value function is used for determining the decomposition path with the least cost to decompose the molecule according to the molecular expression information of the molecule. In the initial stage of decomposition of each first molecule, the cost value function of each first molecule is initialized using the molecular cost value reference information, to avoids the unstable strategy that is likely to be generated due to cost value functions obtained by random initialization, thereby improving the stability of the strategy for determining a molecular decomposition route based on cost value functions.

In an optional implementation, the molecular cost value reference information is a synthetic accessibility score, and the server initializes the cost value function of the molecule by using the synthetic accessibility score. By using the priori advantage of the synthetic accessibility score in representing the ease of synthesis of the molecule, the convergence speed of the molecular cost value function can be improved to a certain extent.

In an optional implementation, when the server allocates the first subtasks to multiple computing nodes, any one of the following manners may be adopted.

In a first optional implementation, the quantity of the first subtasks is the same as the current quantity of computing nodes, and the server allocates the first subtasks to the computing nodes in a one-to-one correspondence.

In a second optional implementation, the quantity of the first subtasks is greater than the current quantity of computing nodes, and the server allocates the first subtasks to the computing nodes according to current computing capabilities of the computing nodes. For example, more than one first subtask is allocated to a computing node with strong computing power, while only one first subtask is allocated to a computing node with weak computing power.

In a third optional implementation, the server allocates the first subtasks according to the quantity of decomposition tasks of first molecules in the first subtask and computing power of the computing nodes. For example, the first subtask including the decomposition tasks of 100 first molecules is assigned to the computing node with strong computing power, and the first subtask including the decomposition tasks of 10 first molecules is assigned to the computing node with weak computing power.

The manner in which the first subtasks are assigned to multiple computing nodes is not specifically limited in the embodiments of this application. Any of the optional methods provided above may be adopted, or any of the optional methods may be combined to obtain a more complex allocation manner.

In step 403, the server receives the first initial cost value functions returned by the computing nodes.

In the embodiments of this application, based on the first initial cost value functions calculated by the computing nodes in the above step 402, the first initial cost value functions corresponding to the first molecules returned by the computing nodes are received.

Steps 401 to 403 are an implementation of the parallel molecular retrosynthetic route exploration process based on a distributed training framework provided in the embodiments of this application. The distributed computing method can speed up the calculation while maintaining the original computing effect, thereby increasing the training speed. In another optional implementation, the server executes the decomposition tasks of the first molecules independently, not based on a distributed training framework. For example, the server obtains the first initial cost value functions of the first molecules based on the molecular expression information of the first molecules and the molecular cost value reference information, which is not specifically limited in the embodiments of this application.

In step 404, for each of the first molecules, in response to complete decomposition of a decomposition level of the first molecule, the server updates the first initial cost value function of the first molecule to obtain a first target cost value function of the first molecule based on a decomposition cost value of a decomposition path of the decomposition level of the first molecule, the first target cost value function is used for determining a decomposition path with a minimum decomposition cost value for the first molecule.

In the embodiments of this application, when the decomposition tasks of the first molecules are executed, a complete decomposition path of a first molecule consists of at least one decomposition level. One decomposition level is used to perform one step of decomposition on a first molecule, that is, the decomposition path of a first molecule consists of at least one step of decomposition.

There are various decomposition methods for each step of decomposition. Each decomposition method corresponds to a decomposition cost value. The decomposition cost value is used for representing the cost required to decompose the first molecule to a current level based on the current decomposition method. When any decomposition level of each first molecule is complete, the server obtains at least one decomposition cost value based on at least one decomposition method existing in the any decomposition level. The server updates the first initial cost value function of each of the first molecules based on the at least one decomposition cost value to obtain a first target cost value function of each of the first molecules based on a decomposition cost value corresponding to any layer of decomposition path of each of the first molecules, the first target cost value function being used for determining a decomposition path with a minimum decomposition cost value for the first molecule.

For example, when the server decomposes the first molecules, there are three decomposition methods $A_1$, $A_2$, and $A_3$ for a first molecule at a first decomposition level $A_1$, that is, when a first step of decomposition is performed on the first molecule. The three decomposition methods correspond to decomposition cost values $a_1$, $a_2$, and $a_3$ respectively. In the case of using cost value reference information to initialize the cost value function, for any composition method, the composition cost value corresponding to the composition method is calculated through the initial cost value function. Based on the cost value reference information as the real value, parameters in the initial cost value function are estimated, and the first initial cost value function is updated, so that the output of the updated cost value function is close to the real value. In this way, it is determined according to the updated cost value function that when the decomposition method $A_1$ is used for assembly, the corresponding decomposition cost value $a_1$ is the smallest. Therefore, the server performs a second decomposition level B on the basis of the decomposition method $A_1$ at the first decomposition level A, that is, performs a second step of decomposition. Similarly, there are three decomposition methods $B_1$, $B_2$, and $B_3$ at the second decomposition level B. The three decomposition methods correspond to decomposition cost values $b_1$, $b_2$, and $b_3$ respectively. The cost value function having been updated based on the decomposition level A is further updated based on these three decomposition cost values, to determine the decomposition method with the smallest decomposition cost value at the decomposition level B. The server sequentially performs a next decomposition level according to the above method, and when any decomposition level is complete, iteratively updates the first initial cost value function to finally obtain the first target cost value function.

In an optional implementation, the first target cost value function is a neural network obtained by deep learning. A constraint condition for determining the decomposition path with the smallest decomposition cost value is called a strategy. When performing the decomposition task for each first molecule, formula (1) is used to calculate a decomposition cost value required by a decomposition path obtained after at least one decomposition level is completed on the first molecule based on this strategy. In this case, different decomposition paths can be obtained based on different decomposition methods, i.e., different decomposition cost values can be obtained. When any decomposition level of the first molecule is complete, a decomposition cost value based on at least one decomposition path can be obtained. Based on the calculated at least one decomposition cost value, the first initial cost value function of the first molecule is updated to obtain a first target cost value function $v_*(m)$ shown in formula (2). In one embodiment, after the first target cost value function is obtained, the strategy is iteratively updated according to formula (3). $\pi(r|m)$ is used as a strategy to decompose molecules, that is, the decomposition paths of the first molecules may be generated based on this strategy. This strategy is iteratively updated to obtain an updated strategy $\pi'(r|m)$. The cost required when the first molecule is decomposed according to the updated strategy $\pi'(r|m)$ is the smallest. Formulas (1) to (3) are as follows:

$$c_{tot} = \sum_r c_{rxn}(r) \qquad (1)$$

In formula (1), r represents a reaction that can be selected, that is, a decomposition method existing in the decomposition path of the first molecule, $c_{tot}$ represents the total decomposition cost required to decompose the first molecule, and $c_{rxn}$ represents the cost value when the first molecule is decomposed in a decomposition level according to the reaction r.

$$v_*(m) = \min_r[c_{rxn}(r) + \sum_{m' \in M(r)} v_*(m')] \qquad (2)$$

In formula (2), m represents the product, that is, the first molecule, and M(r) represents a set of molecules. $v_*(m')$ is a cost value of the product m obtained by decomposing the first molecule according to r reaction. For example, the obtained $v_*(m')$ may be 1 which represents easy in decomposition. In deep learning, m' is a next state of a current state. $v_*(m')$ is a value obtained by the cost value function.

It is to be understood that, according to the principle of deep learning, the value of performing action a in state t is equal to the sum of the immediate reward after performing action a in state t plus the value of all possible next states. In the molecular decomposition task in this embodiment, "molecule m" is equivalent to "state t", "reaction r" is equivalent to "action a", Crxn is equivalent to "immediate reward after performing action a in state t", the "product m" is equivalent to the "next state". The value obtained by adding the summation of $v_*(m')$ and Crxn is a cost value, which refers to the cost value required to decomposing the molecule m according to the reaction r, where r refers to any reaction, and Crxn refers to the cost value for decomposing the molecule according to reaction r in a composition level, which is the immediate reward after performing action a in state t in deep learning. The summation of $v_*(m')$ refers to the cost value of all possible products m' obtained by decomposing the molecule m according to reaction r, which corresponds to "the sum of the values of all possible next states" in deep learning. In the embodiment of this application, solving the minimum value is to determine the decomposition method corresponding to the minimum decomposition cost value of the molecule m.

$$\pi'(r|m) = \begin{cases} 1 \text{ if } r = \arg\min_{r\in\mathcal{R}(m)}\left[c_{\text{rxn}}(r) + \sum_{m'\in\mathcal{M}(r)} v_{\pi}(m')\right] \\ 0 \text{ otherwise} \end{cases} \tag{3}$$

In formula (3), $v_{\pi}(m')$ represents a cost value function for expanding the first molecule according to $\pi$ to obtain the product m. The summation of $v_{\pi}(m')$ refers to the cost value of all possible products m' obtained by decomposing the molecule m according to reaction r.

Formula (3) is an Iverson bracket used to update the strategy. $\pi'(r|m)$ represents a strategy, this strategy is to decomposing the molecule m according to reaction r. If this reaction r is a reaction corresponding to the minimum cost value for decomposing the molecule m, $\pi'(r|m)$ is equal to 1, indicating that the strategy is feasible, otherwise $\pi'(r|m)$ is equal to 0.

Value iteration in deep learning is used for updating the strategy in the above formula (3). In the value iteration in deep learning, after the value function is updated, it is considered that the value function can well estimate whether each state is good or bad. In this case each state is traversed to select the action with the highest value under the current state, and this action is performed after this. That is, action is selected based on the state value. In the embodiment of the present application, it is considered that the updated cost value function can well evaluate reaction r with the minimum cost value to decomposing the molecule m, thus the reaction r with the minimum decomposition cost value can be selected. The selected reaction r is the obtained molecular retrosynthesis strategy.

In the embodiment, if the decomposition method corresponding to the minimum decomposition cost value of molecule m is determined by formula (2), then the strategy $\pi'(r|m)$ =1, which indicates that the strategy is feasible. Otherwise $\pi'(r|m)$ =0.

In one embodiment, the cost value function may be initialized by using molecular cost value reference information. The molecular cost value reference information may be a Synthetic Accessibility score. For the cost value function shown in the formula (2), the difficulty in decomposing a molecule can be predicted according to the Synthetic Accessibility score to obtain the Synthetic Accessibility score of the molecule. For example, the Synthetic Accessibility score of the molecule is predicted to be 10, then 10 is taken as the real value, and the parameters in the cost value function in formula (2) are estimated to cause the value output by the cost value function to be close to 10. That is, using the Synthetic Accessibility score as an empirical reference value, to initialize the parameters in the cost value function, so that the difference between the output result of the cost value function and the real value in the subsequent training process is relatively small. The convergence speed of the molecular cost value function during training is improved by initializing the parameters in the cost value function using the Synthetic Accessibility score.

In an optional implementation, when the server performs the decomposition task for each first molecule, there are K decomposition levels, where K is greater than or equal to 1. Each time a decomposition level is completed, the corresponding decomposition cost value is 1. When a molecule obtained after decomposition is performed according to at least one decomposition method existing in any decomposition level is a molecule existing in the molecule database, the decomposition cost value of the current decomposition method is 0. When the molecule obtained is an impossible molecule, for example, when the molecule does not exist in the molecule database, the decomposition cost value of the current decomposition method is 100. The server calculates the decomposition cost value of each first molecule based on any decomposition level based on the above formula (1). Finally, the server can update the first initial cost value function of the first molecule based on the decomposition cost value obtained through any decomposition level, so as to obtain the first target cost value function.

In an optional implementation, the server performs the decomposition task for each first molecule based on the molecular retrosynthesis reference information. The molecular retrosynthesis reference information is used for providing any decomposition level of each first molecule with at least one decomposition method based on the decomposition level. For example, the molecular retrosynthesis reference information is a computer-aided retrosynthesis method based on molecular similarity. This method can provide available decomposition schemes for molecules. For another example, the molecular retrosynthesis reference information is a template neural network, which is used to provide at least one reaction template, that is, a decomposition method, for the decomposition of the molecule. The reaction template is used to describe the process of a type of chemical reaction, including the breaking of an existing chemical bond and the formation of a new chemical bond.

In an optional implementation, in the above process of decomposing each first molecule, the decomposing process of each first molecule is modeled as an expanding game. Using $\pi(r|m)$ as a strategy for decomposing the molecules, where m represents the product and r represents a reaction that can be selected, the cost value function of the current molecule can be obtained. During the expansion process, the first target cost value function $v_*(m)$ is continuously updated to determine the decomposition path with the smallest decomposition cost. The strategy is iteratively updated at each round of expansion.

In step 405, in response to a decomposition task of any one of the first molecules satisfying a target decomposition condition, the server determines a first decomposition path of the first molecule based on the first target cost value function of the first molecule.

In the embodiments of this application, the target decomposition condition is that a path depth of a decomposition path, of each first molecule, obtained in at least one decomposition level is less than a target depth and there is no method for decomposing the molecules obtained from the decomposition path, or is that the path depth of the decomposition path of each first molecule obtained in at least one decomposition level is equal to the target depth. The target depth is a depth threshold preset by the server. For example, the depth threshold is set to 5.

The following is an example where the target depth is 5:

If a path depth of a decomposition path obtained after P decomposition levels are performed for a first molecule is less than 5, where P is greater than or equal to 1 and less than 5, and there is no further decomposition method for the obtained molecules, that is, the obtained molecules are all molecules that can be obtained from the molecule database, then the decomposition task of the first molecule satisfies the target decomposition condition, and the server obtains a first target cost value function of the first molecule based on the current decomposition level, and determines a decomposition path with the smallest decomposition cost value based on this function, where the decomposition path is the first decomposition path of the first molecule.

If a path depth of a decomposition path obtained after Q decomposition levels are performed for a first molecule, where Q is equal to 5, the decomposition task of the first molecule satisfies the target decomposition condition, and the server obtains a first target cost value function of the first molecule based on the current decomposition level, and determines a decomposition path with the smallest decomposition cost value based on this function, where the decomposition path is the first decomposition path of the first molecule.

In step 406, the server determines cost value information of each of the first molecules based on the first decomposition path of each of the first molecules.

In the embodiments of this application, the cost value information of each first molecule is used for representing a cost required to decompose the first molecule according to the corresponding first decomposition path. In an optional implementation, the server determines the cost value information corresponding to each first molecule based on the first target cost value function of each first molecule, that is, v,(m). In another optional implementation, during decomposition of each first molecule, each time any decomposition level is completed, a decomposition path with the minimum decomposition cost value based on the current decomposition level can be obtained, that is, the cost value information corresponding to each first molecule based on the current decomposition level can be obtained.

In step 407, the server obtains a first cost dictionary according to the molecular expression information of each of the first molecules and the cost value information of each of the first molecules, the first cost dictionary including the molecular expression information of each of the first molecules and the cost value information of each of the first molecules.

In the embodiments of this application, the molecular expression information and the corresponding cost value information in the first cost dictionary exist in a one-to-one correspondence manner. For example, a key-value pair may be used in the first cost dictionary to represent the molecular expression information of each molecule and the cost value information corresponding to each molecule. That is, there are N key-value pairs in the first cost dictionary, where N is greater than or equal to 1: {$Smile_1$:$cost_1$}; {$Smile_2$:$cost_2$}...{$Smile_N$:$cost_N$}, which are respectively used for representing molecule 1 to molecule N and cost value information corresponding to the molecules.

By performing the above steps 401 to 407, the server completes the exploration of the upper-layer retrosynthetic routes of multiple first molecules, and obtains a first cost dictionary corresponding to the upper-layer retrosynthetic routes.

In step 408, the server determines molecular expression information of at least one second molecule based on the first decomposition paths of the first molecules, each of the second molecules is a molecule that can be decomposed into obtainable molecules.

In the embodiments of this application, after each first molecule is decomposed based on the corresponding first decomposition path, multiple molecules can be obtained. Among the multiple molecules, some molecules can be further decomposed, and there is a decomposition path. These molecules are determined as second molecules.

In an optional implementation, in performing a decomposition task of a first molecule, the server decomposes the first molecule based on the first decomposition path of the first molecule to obtain at least one molecule, and then determines molecular expression information of the obtained at least one molecule. Based on the molecular expression information, the server determines whether the obtained molecule can be further decomposed. If the molecule can be further decomposed, it is determined whether there is a decomposition method for this molecule, and if there is a decomposition method for this molecule, this molecule is determined as a second molecule, that is, this molecule is determined as an obtainable molecule.

In the embodiments of this application, according to the sequence of the above steps 406 to 408, the server first determines the cost value information of each first molecule based on the first decomposition path of each first molecule to obtain the first cost dictionary, and then determines at least one second molecule. In another optional implementation, the server may first determine at least one second molecule based on the first decomposition path of each first molecule according to step 408, and then execute steps 406 and 407 to obtain the first cost dictionary. This is not specifically limited in the embodiments of this application.

In step 409, the server clusters the second molecules to obtain a plurality of sets, each of the sets including at least one second molecule with similar molecular structure.

In the embodiments of this application, clustering means a process of grouping the second molecules into multiple sets based on structural similarities between the molecules. The second molecules in each set have similar molecular structures. In one embodiment, the server is capable of classifying the at least one second molecule based on other classification methods, e.g., a Bayesian classification algorithm or the like. This is not specifically limited in the embodiments of this application.

In an optional implementation, the server clusters the second molecules based on a Taylor Butina (TB) algorithm. The structural similarity between two second molecules is determined by a Tanimoto coefficient, as shown in formula (4):

$$T = \frac{m_i \cdot m_j}{|m_i|^2 + |m_j|^2 - m_i \cdot m_j} \qquad (4)$$

In formula (4), $m_i$ represents an ECPF4 molecular fingerprint of molecule i, and $m_j$ represents an ECPF4 molecular fingerprint of molecule j. For example, the ECPF4 molecular fingerprint has a fingerprint length of 1024 and a radius of 3.

When the Tanimoto coefficient between two molecules is less than a preset threshold, it is determined that the two molecules belong to the same class of molecules, and the two molecules are put into the same set. For example, the preset threshold is 0.4. The setting of the preset threshold is not specifically limited in the embodiments of this application and may be adjusted according to actual situations.

In the embodiments of this application, the at least one second molecule is clustered using the TB algorithm. In another optional implementation, the server can cluster the second molecules using other clustering algorithms, for example, k-means clustering algorithm, mean-shift clustering algorithm, etc. This is not specifically limited in the embodiments of this application.

In step 410, the server determines cluster centers of the sets as a plurality of third molecules, each of the third molecules being a representative molecule in the set to which the third molecule belongs.

In the embodiments of this application, the cluster centers are the centers of the plurality of sets generated by clustering the second molecules, the center is a representative second molecule in the corresponding set, and the representative second molecule is determined as the third molecule.

After exploring the upper-layer retrosynthetic routes of the first molecules by performing the above steps 408 to 410, the server screens a plurality of obtained molecules that can be further decomposed and for which there is still a decomposition method, to obtain a plurality of representative third molecules. These third molecules are used as starting molecules of lower-layer retrosynthetic routes, so that during the exploration of the retrosynthetic route of each molecule, it is not necessary to continuously perform decomposition until the maximum depth is reached or until the exploration of the entire route is completed, thereby saving time. In the process of layering, the use of representative molecules as the third molecules greatly reduces the calculation amount of the subsequent training process, reduces the time for determining a molecular retrosynthetic route, and further improves the training speed.

In step 411, the server obtains a second cost dictionary based on second decomposition paths of the third molecules, the second cost dictionary includes the molecular expression information of each of the third molecules and cost value information of each of the third molecules, and the cost value information of each third molecule representing a cost value required to decompose the third molecule according to the corresponding second decomposition path.

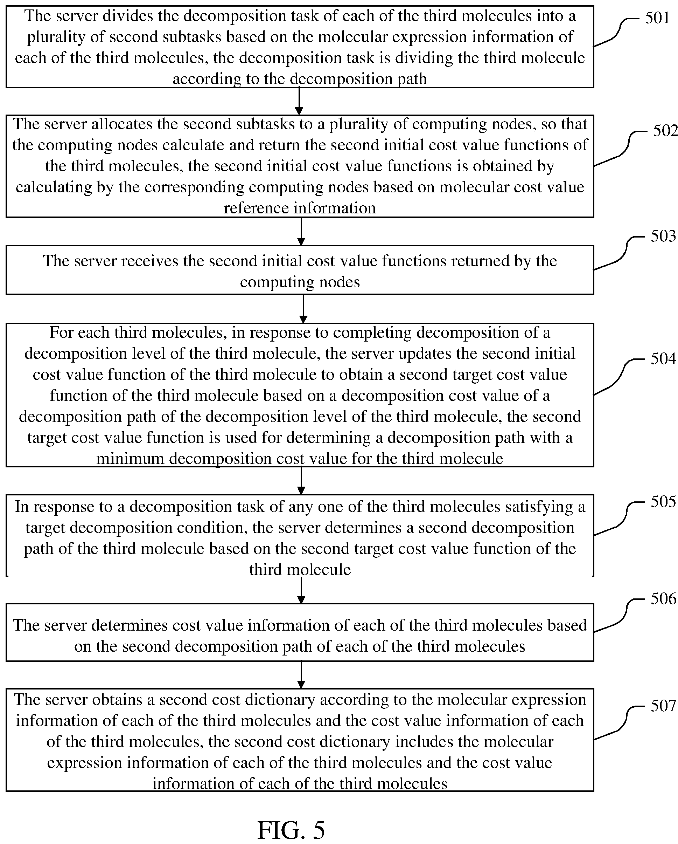

In the embodiments of this application, reference is made to FIG. 5 for the process of obtaining the second cost dictionary based on the second decomposition paths of the third molecules. FIG. 5 is a flowchart of a method for obtaining a second cost vocabulary according to an embodiment of this application. Specifically, the method includes the following steps 501 to 507. Steps 501 to 507 are similar to the process of executing the above steps 401 to 407, so steps 501 to 507 are merely briefly described below, and the specific optional implementations will not be repeated.

In step 501, the server divides the decomposition task of each of the third molecules into a plurality of second subtasks based on the molecular expression information of each of the third molecules, the decomposition task is dividing the third molecule according to the decomposition path.

In the embodiments of this application, the third molecules are molecules obtained after performing the above step 410. The server divides the decomposition tasks of the plurality of third molecules into multiple subtasks based on the molecular expression information of each third molecule.

In step 502, the server allocates the second subtasks to a plurality of computing nodes, so that the computing nodes calculate and return the second initial cost value functions of the third molecules, the second initial cost value functions is obtained by calculating by the corresponding computing nodes based on molecular cost value reference information.

In the embodiments of this application, the server can process data based on multiple computing nodes at the same time. The server assigns multiple second subtasks to multiple computing nodes, with one second computing node being responsible for the decomposition task of at least one third molecule. The second initial cost value function is an initial cost value function of each third molecule calculated by the computing node based on the molecular expression information of each third molecule and by using the molecular cost value reference information. The cost value function is used for determining the decomposition path with the least cost to decompose the molecule according to the molecular expression information of the molecule. In the initial stage of decomposition of each third molecule, the cost value function of each third molecule is initialized using the molecular cost value reference information, to avoids the unstable strategy that is likely to be generated due to cost value functions obtained by random initialization, thereby improving the stability of the strategy for determining a molecular decomposition route based on cost value functions.

In step 503, the server receives the second initial cost value functions returned by the computing nodes.

In the embodiments of this application, based on the second initial cost value functions calculated by the computing nodes in the above step 502, the server can receive the second initial cost value functions corresponding to the third molecules returned by the computing nodes.

In step 504, for each of the third molecules, in response to completing decomposition of a decomposition level of the third molecule, the server updates the second initial cost value function of the third molecule to obtain a second target cost value function of the third molecule based on a decomposition cost value of a decomposition path of the decomposition level of the third molecule, the second target cost value function is used for determining a decomposition path with a minimum decomposition cost value for the third molecule.

In the embodiments of this application, when the decomposition tasks of the third molecules are executed, a complete decomposition path of a third molecule consists of at least one decomposition level. One decomposition level is used to perform one step of decomposition on a third molecule, that is, the decomposition path of a third molecule consists of at least one step of decomposition.

There are various decomposition methods for each step of decomposition. Each decomposition method corresponds to a decomposition cost value. The decomposition cost value is used for representing the cost required to decompose the third molecule to a current level based on the current decomposition method. When any decomposition level of each third molecule is complete, the server obtains at least one decomposition cost value based on at least one decomposition method existing in the any decomposition level. The server updates the second initial cost value function of each of the third molecules based on the at least one decomposition cost value to obtain a second target cost value function of each of the third molecules based on a decomposition cost value corresponding to any layer of decomposition path of each of the third molecules, the second target cost value function being used for determining a decomposition path with a minimum decomposition cost value for the third molecule.

In step 505, in response to a decomposition task of any one of the third molecules satisfying a target decomposition condition, the server determines a second decomposition path of the third molecule based on the second target cost value function of the third molecule.

In the embodiments of this application, the target decomposition condition means that a path depth of a decomposition path obtained for each third molecule based on at least one decomposition level is less than a target depth and there is no decomposition method for the obtained molecules, or means that the path depth of the decomposition path obtained for each third molecule based on at least one decomposition level is equal to the target depth. The target depth is a depth threshold preset by the server. For example, the depth threshold is set to 5. The second decomposition path refers to a path that requires the least cost to decompose the third molecule until the target decomposition condition is met.

In step 506, the server determines cost value information of each of the third molecules based on the second decomposition path of each of the third molecules.

In the embodiments of this application, the cost value information of each third molecule is used for representing a cost value required to decompose the third molecule according to the corresponding second decomposition path.

In step 507, the server obtains a second cost dictionary according to the molecular expression information of each of the third molecules and the cost value information of each of the third molecules, the second cost dictionary includes the molecular expression information of each of the third molecules and the cost value information of each of the third molecules.

In the embodiments of this application, the molecular expression information and the corresponding cost value information in the second cost dictionary exist in a one-to-one correspondence manner. For example, a key-value pair may be used in the second cost dictionary to represent the molecular expression information of each molecule and the cost value information corresponding to each molecule. That is, there are M key-value pairs in the second cost dictionary, where M is greater than or equal to 1: $\{Smile_1:cost_1\}$; $\{Smile_2:cost_2\}$...$\{Smile_M:cost_M\}$, which are respectively used for representing molecule 1 to molecule M and cost value information corresponding to the molecules.

By performing the above steps 501 to 507, the server completes the exploration of the lower-layer retrosynthetic routes, and obtains a second cost dictionary corresponding to the lower-layer retrosynthetic routes. By such a layering method, the exploration of the entire retrosynthetic routes of multiple first molecules is layered. The exploration of the upper-layer retrosynthetic routes is completed first, and after representative molecules are selected, the exploration of the lower-layer retrosynthetic routes is completed. Whereby, the time required for exploring molecular retrosynthetic routes is greatly reduced.

In step 412, the server trains a second neural network based on the molecular expression information and the corresponding cost value information of each molecule in the second cost dictionary.

In the embodiments of this application, the neural network includes a first neural network and a second neural network. The first neural network is a neural network obtained by training based on information in the first cost dictionary. The second neural network is a neural network obtained by training based on information in the second cost dictionary. Step 412 includes the following steps: the server inputs the molecular expression information of a molecule in the second cost dictionary into the second neural network, and performs calculation based on a network parameter of the second neural network to obtain predicted cost value information corresponding to the molecule; the server determines a model loss of the second neural network based on the predicted cost value information corresponding to each molecule and the cost value information corresponding to each molecule in the second cost dictionary; and the server adjusts the network parameter in the second neural network according to the model loss of the second neural network, inputs the molecular expression information of a new molecule again based on the adjusted second neural network, and iteratively adjusts the network parameter in the second neural network according to the predicted cost value information obtained by each input and the cost value information corresponding to the inputted molecule, until the model loss of the second neural network meets a target condition; then the server determines the current second neural network as the trained second neural network.

The process of training the second neural network in the embodiments of this application may further include other steps, which will not be detailed here.

In step 413, the server updates the first cost dictionary based on the second cost dictionary to obtain an updated first cost dictionary.

In the embodiments of this application, the third molecules in the second cost dictionary are determined by clustering the plurality of molecules obtained by decomposing the first molecule in the first cost dictionary. The second cost dictionary includes the molecule expression information of each third molecule and the cost value information corresponding to each third molecule. The server uses the molecule expression information of each third molecule and the cost value information corresponding to each third molecule to update the cost value information of each first molecule, to obtain the updated first cost dictionary. Through the bottom-up update process of the cost value information of each first molecule by the server, the accuracy of determining the cost of molecular retrosynthesis is improved.

In an optional implementation, the server updates the first cost dictionary based on the second cost dictionary by the following specific process: determining the molecular expression information of the first molecules respectively corresponding to the third molecule according to the molecular expression information of the third molecules, and then summing up the cost value information of each third molecule in the second cost dictionary and the cost value information of the corresponding first molecule in the first cost dictionary to obtain updated cost value information of the first molecule, that is, to obtain the updated first cost dictionary. For example, according to the cost value information of a third molecule, the server obtains that the minimum decomposition cost for decomposing the third molecule is 10, and the minimum decomposition cost of the corresponding first molecule is 20. In this case, the updated minimum decomposition cost of the first molecule is 30. The manner of updating the first cost dictionary is not limited in the embodiments of this application.

In step 414, the server trains the first neural network based on the molecular expression information and the cost value information of each molecule in the updated first cost dictionary.

In an embodiment of this application, step 414 includes the following steps: the server inputs the molecular expression information of a molecule in the first cost dictionary into the first neural network, and performs calculation based on a network parameter of the first neural network to obtain predicted cost value information corresponding to the molecule; the server determines a model loss of the first neural network based on the predicted cost value information corresponding to each molecule and the cost value information corresponding to each molecule in the first cost dictionary; and the server adjusts the network parameter in the first neural network according to the model loss of the first neural network, inputs the molecular expression information of a new molecule again based on the adjusted first neural network, and iteratively adjusts the network parameter in the first neural network according to the predicted cost value information obtained by each input and the cost value information corresponding to the inputted molecule, until the model loss of the first neural network meets a target condition; then the server determines the current first neural network as the trained first neural network.

The process of training the first neural network in the embodiments of this application may further include other steps, which will not be detailed here.

In step 415, the server combines the trained second neural network and the trained first neural network to obtain the target neural network.

In the embodiments of this application, the target neural network is configured to output cost value information corresponding to a target molecule according to input molecular expression information of the target molecule. The server combines the trained second neural network and the trained first neural network to obtain the target neural network. For example, the server obtains the target neural network in a serial manner. For the target molecule, the server inputs the molecular expression information of the target molecule into the first neural network, and outputs the cost value information of the target molecule based on the upper-layer retrosynthetic route. Then, the server determines molecules for which there is a further decomposition method among molecules obtained after exploration of the upper-layer retrosynthetic route of the target molecule, inputs the molecules for which there is a further decomposition method into the second neural network, outputs the cost value information of the target molecule based on the lower-layer retrosynthetic route, and finally obtains the complete retrosynthetic route of the target molecule. The manner of obtaining the target neural network is not specifically limited in the embodiments of this application.

By performing the above steps 412 to 415, the server performs training based on the second cost dictionary and the updated first cost dictionary to obtain the target neural network. This can maximize the generalization ability of the target neural network of this application for molecules that do not participate in the training process, so that a decomposition path can be obtained for any molecule based on the target neural network.

In the embodiments of this application, according to the sequence of the above steps 412 to 415, the server first trains the second neural network, then updates the first cost dictionary, and trains the first neural network. In another optional implementation, the server may first execute step 413 to update the first cost dictionary, and then execute steps 412, 414, and 415 to obtain the target neural network. This is not specifically limited in the embodiments of this application.

In the embodiments of this application, the above steps 412 to 415 are an implementation of the process of obtaining the target neural network through training according to the embodiments of this application. In an embodiment, the server can also obtain the target neural network through other training methods, which is not specifically limited in the embodiments of this application.

In addition, referring to Table 2, Table 2 shows a comparison of experimental effects of related methods and the embodiments of this application. In the experiment, the time required for successfully decomposing the same number of molecules based on a standard data set is obtained. In contrast, compared with the related methods, the method provided in the embodiments of this application requires a significantly shorter time in decomposing the same number of molecules than those required by the other methods.

Table 2

| Algorithm\Number of molecules successfully decomposed | 1000 | 2000 | 3000 |
|---|---|---|---|
| Monte Carlo Tree (MCTS) | 55h | 98h | -- |
| Reinforcement learning | 52h | 107h | 146h |
| Distributed reinforcement learning | 23h | 45h | 63h |
| Embodiment of this application | 12h | 25h | 35h |

Referring to Table 3, Table 3 shows a comparison of experimental effects of related methods and the embodiments of this application. In the experiment, a decomposition result of decomposing the same number of molecules based on a standard data set is obtained. The experimental results show that compared with related methods, when decomposing the same number of molecules, the method provided in the embodiments of this application can successfully decompose a larger number of molecules, with a smaller number of molecules failing to be decomposed and a smaller number of failures due to an excessively large number of decomposition layers.

Table 3

| Algorithm\Decomposition Result | Number of successful decompositions | Number of failures due to inability to decompose | Number of failures due to excessively large number of decomposition layers |
|---|---|---|---|
| Monte Carlo Tree (MCTS) | 2567 | 574 | 698 |
| Reinforcement learning | 3122 | 194 | 523 |
| Distributed reinforcement learning | 3085 | 289 | 465 |

(continued)

| Algorithm\Decomposition Result | Number of successful decompositions | Number of failures due to inability to decompose | Number of failures due to excessively large number of decomposition layers |
| --- | --- | --- | --- |
| Embodiment of this application | 3578 | 65 | 196 |

Certainly, the above different implementations may be combined with each other to form different embodiments, which is not limited in the embodiments of this application.

In the embodiments of this application, a training method for a neural network for determining a molecular retrosynthetic route is provided. When a retrosynthetic route of each of a plurality of molecules is determined, a concept of hierarchical learning is adopted. A training process of a molecular retrosynthetic route requiring deeper exploration is split into multiple layers for training to accelerate the training, and the complete retrosynthetic reaction process is replaced by multiple layers of molecular retrosynthetic routes. After the training of one layer of molecular retrosynthesis route is completed, a representative molecule is selected by molecular screening and used as a starting molecule in a next layer of molecular retrosynthetic route, which effectively improves the exploration efficiency of the molecular retrosynthetic route, whereby accurate molecular cost information is more efficiently extracted. The hierarchical approach greatly reduces the computational overhead brought about by determining the molecular retrosynthetic route, and reduces the time for determining the molecular retrosynthetic route while ensuring the accuracy of the molecular retrosynthetic route.

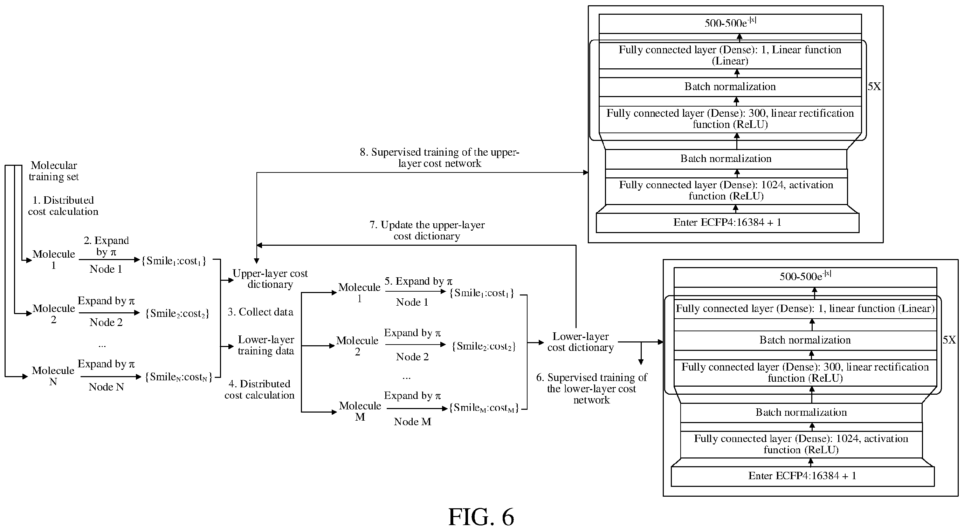

In the following, an example of a training method for a neural network for determining a molecular retrosynthetic route provided in the embodiments of this application is described with reference to an actual situation. As shown in FIG. 6, FIG. 6 is an architecture diagram of a training method for a neural network for determining a molecular retrosynthetic route according to an embodiment of this application. An example where the process of constructing a molecular retrosynthetic route is divided into two layers is described.

First, for molecules in a molecular training set, a distributed training framework is used to perform parallel exploration of upper-layer retrosynthetic routes, and all molecular decomposition tasks are allocated to N computing nodes, where N is greater than or equal to 1. The computing nodes perform the decomposition tasks of the molecules respectively. In the early stage of decomposition of each molecule, the cost value function is initialized using a molecular synthetic accessibility score. By using the priori advantage of the synthetic accessibility score in representing the ease of synthesis of the molecule, the convergence speed of the molecular cost value function is improved. Then according to the strategy $\pi(r|m)$, each molecule is expanded, and finally the cost of each molecule under the corresponding strategy is obtained. When the calculation performed by each computing node is complete, the molecular expression information and cost value information of all molecules are summarized to obtain an upper-layer cost dictionary.

Secondly, after the exploration of the upper-layer retrosynthetic routes is complete, molecules that can be further decomposed and for which there is still a decomposition method among the obtained molecules are collected, and clustered using the TB algorithm. Representative molecules are selected as an initial training set for the exploration of lower-layer molecular retrosynthetic routes, that is, as lower-layer training data. The molecules in the lower-layer molecular training set are expanded by a molecule expanding process similar to that for the upper layer to obtain a lower-layer cost dictionary.

Thirdly, the upper-layer cost dictionary is updated based on the lower-layer cost dictionary, the corresponding neural networks are trained based on the two cost dictionaries respectively, and supervised training of the two neural networks is performed using deep learning technology to obtain the target neural network that can explore retrosynthetic routes of new target molecules. The process of supervised training of the neural network is as follows: Molecular expression information of a molecule is inputted, and processed by a first fully connected layer (Dense), followed by batch normalization processing of the data. Then the data is processed by a second fully connected layer, followed by batch normalization processing. The process of processing by a second fully connected layer followed by batch normalization processing of the data is repeated five times. Finally, $500-500e^{-|x|}$ is outputted through a third fully connected layer, where x is a variable.

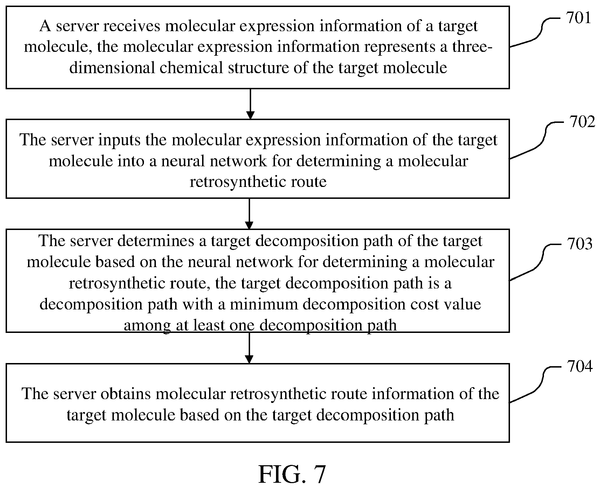

FIG. 7 is a flowchart of a method for determining a molecular retrosynthetic route according to an embodiment of this application. As shown in FIG. 7, this embodiment of this application is described using an example where the method is applied to a server. The method includes the following steps:

In step 701, a server receives molecular expression information of a target molecule, the molecular expression information represents a three-dimensional chemical structure of the target molecule.

In the embodiments of this application, the target molecule is a molecule that cannot be obtained from a molecule database. The server receives the molecular expression information of the target molecule. For example, the server receives a simplified molecular input line entry specification of the target molecule, that is, a string of characters used for representing a three-dimensional chemical structure of the molecule.

In step 702, the server inputs the molecular expression information of the target molecule into a neural network for determining a molecular retrosynthetic route.

In the embodiments of this application, after receiving the molecular expression information of the target molecule, the server inputs same into the neural network for determining a molecular retrosynthetic route that is provided in the embodiments of this application.

In step 703, the server determines a target decomposition path of the target molecule based on the neural network for determining a molecular retrosynthetic route, the target decomposition path is a decomposition path with a minimum decomposition cost value among at least one decomposition path.

In the embodiments of this application, based on the neural network for determining a molecular retrosynthetic route that is provided in the embodiments of this application, cost value information corresponding to the target molecule is outputted. A decomposition path corresponding to the cost value information is a decomposition path with the lowest decomposition cost value among all possible decomposition paths of the target molecule.

In an optional implementation, step 703 includes the following steps:

the neural network for determining a molecular retrosynthetic route including a first neural network and a second neural network, outputting cost value information of an upper-layer retrosynthetic route of the target molecule based on the first neural network; determining a first decomposition path of the target molecule, a path depth of the first decomposition path being less than or equal to a target depth; determining molecular expression information of a molecule for which a further decomposition method exists among molecules obtained by decomposing the target molecule based on the first decomposition path; inputting the molecular expression information of the molecule for which a further decomposition method exists into the second neural network; outputting cost value information of a lower-layer retrosynthetic route of the target molecule based on the second neural network; determining a second decomposition path of the target molecule; and determining the target decomposition path of the target molecule based on the first decomposition path and the second decomposition path.

In step 704, the server obtains molecular retrosynthetic route information of the target molecule based on the target decomposition path.

In the embodiments of this application, according to the target decomposition path obtained in step 703, a decomposition reaction of the target molecule based on each step of the decomposition path is obtained, and a complete retrosynthetic route of the target molecule is finally determined.

In the embodiments of this application, a method for determining a molecular retrosynthetic route is provided. Through a neural network for determining a molecular retrosynthetic route, the retrosynthetic route of a target molecule is obtained, with a short time and high accuracy.

Although the steps in the flowcharts of the embodiments are displayed sequentially according to instructions of arrows, these steps are not necessarily performed sequentially according to a sequence instructed by the arrows. Unless otherwise clearly specified in this specification, the steps are performed without any strict sequence limit, and may be performed in other sequences. In addition, at least some steps in the flowcharts in the foregoing embodiments may include a plurality of steps or a plurality of stages. The steps or the stages are not necessarily performed at the same moment, but may be performed at different moments. The steps or the stages are not necessarily performed in sequence, but may be performed in turn or alternately with another step or at least some of steps or stages of the another step.

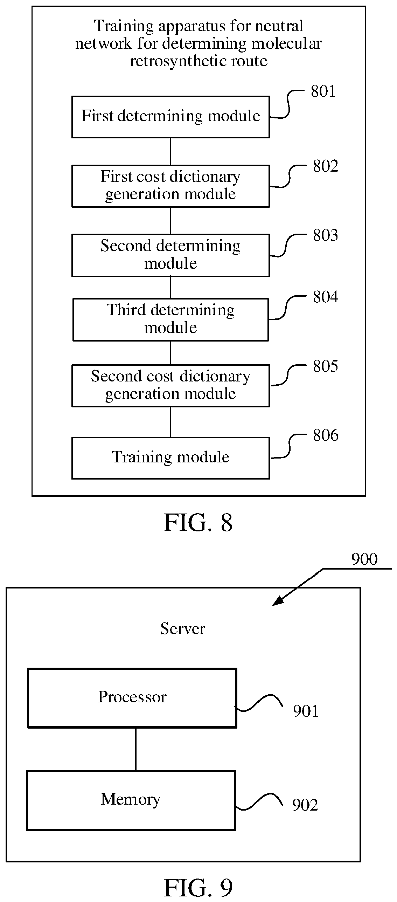

FIG. 8 is a block diagram of a training apparatus for a neural network for determining a molecular retrosynthetic route according to an embodiment of this application. The apparatus is configured to execute the steps of the training method for a neural network for determining a molecular retrosynthetic route. Referring to FIG. 8, the apparatus includes: a first determining module 801, a first cost dictionary generation module 802, a second determining module 803, a third determining module 804, a second cost dictionary generation module 805, and a training module 806.

The first determining module 801 is configured to determine a first decomposition path of each of a plurality of first molecules based on molecular expression information of the plurality of first molecules, a path depth of the first decomposition path being less than or equal to a target depth.

The first cost dictionary generation module 802 is configured to obtain a first cost dictionary based on the first decomposition paths of the first molecules, the first cost dictionary including the molecular expression information of each of the first molecules and cost value information corresponding to each of the first molecules, and the cost value information of each first molecule representsa cost required to decompose the first molecule according to the corresponding first decomposition path.

The second determining module 803 is configured to determine molecular expression information of at least one second molecule based on the first decomposition paths of the first molecules, each of the second molecules being a molecule that can be decomposed into obtainable molecules.

The third determining module 804 is configured to determine a plurality of third molecules from the second molecules, each of the third molecules represents a class of the second molecules.

The second cost dictionary generation module 805 is configured to obtain a second cost dictionary based on a second decomposition path of each of the third molecules, the second cost dictionary including the molecular expression information of each of the third molecules and cost value information corresponding to each of the third molecules, and the cost value information of each third molecule represents a cost required to decompose the third molecule according to the corresponding second decomposition path.

The training module 806 is configured to perform training based on the first cost dictionary and the second cost dictionary to obtain a target neural network, the target neural network being configured to output cost value information corresponding to a target molecule according to input molecular expression information of the target molecule.

In an optional implementation, the first determining module 801 includes:

an obtaining unit, configured to obtain a first initial cost value function of each of the first molecules based on the molecular expression information and cost value reference information of each of the first molecules;

an updating unit, configured to, in response to completing decomposition of a decomposition level of each of the first molecules, update the first initial cost value function of each of the first molecules to obtain a first target cost value function of each of the first molecules based on a decomposition cost value corresponding to a decomposition path of the decomposition level of the respective first molecule, the first target cost value function being used for determining a decomposition path with a minimum decomposition cost value for the first molecule; and

a determining unit, configured to, in response to a decomposition task of any one of the first molecules satisfying a target decomposition condition, determine a first decomposition path of the first molecule based on the first target cost value function of the first molecule.

In an optional implementation, the obtaining unit is configured to execute operations of:

dividing the decomposition task of each of the first molecules into a plurality of first subtasks based on the molecular expression information of the respective first molecules, the decomposition task being dividing the first molecule according to the decomposition path;

allocating the first subtasks to a plurality of computing nodes, so that the computing nodes calculate and return the first initial cost value functions of the first molecules, the first initial cost value functions being obtained by calculating by the corresponding computing nodes based on molecular cost value reference information, and the molecular cost value reference information representing synthetic accessibility of the molecule; and

receiving the first initial cost value functions returned by the computing nodes.

In an optional implementation, the first cost dictionary generation module 802 is configured to execute operations of:

determining cost value information corresponding to each of the first molecules based on the first decomposition path of the respective first molecules; and

obtaining the first cost dictionary according to the molecular expression information of each of the first molecules and the cost value information corresponding to each of the first molecules.

In an optional implementation, the third determining module 804 is configured to execute operations of:

clustering the second molecules to obtain a plurality of sets, each of the sets including at least one second molecule with a similar molecular structure; and

obtaining the plurality of third molecules by respectively determining a cluster center of each of the sets as the third molecule corresponding to the set, each of the third molecules being a representative molecule in the set to which the respective third molecule belongs.

In an optional implementation, the training module 806 includes:

a first training unit, configured to train a second neural network based on the molecular expression information and the corresponding cost value information of each molecule in the second cost dictionary;

a second cost dictionary updating unit, configured to update the first cost dictionary based on the second cost dictionary to obtain an updated first cost dictionary;

a second training unit, configured to train the first neural network based on the molecular expression information and the corresponding cost value information of each molecule in the updated first cost dictionary; and

a combining unit, configured to combine the trained second neural network and the trained first neural network to obtain the target neural network.

In an optional implementation, the first training unit is configured to execute operations of:

inputting the molecular expression information of each molecule in the second cost dictionary into the second neural network to obtain predicted cost value information corresponding to each molecule;

determining a model loss of the second neural network based on the predicted cost value information corresponding to each molecule and the cost value information corresponding to each molecule in the second cost dictionary; and

adjusting a network parameter in the second neural network according to the model loss of the second neural network.

In the embodiments of this application, a training apparatus for a neural network for determining a molecular retrosynthetic route is provided. When a retrosynthetic route of each of a plurality of molecules is determined, a concept of hierarchical learning is adopted. A training process of a molecular retrosynthetic route requiring deeper exploration is split into multiple layers for training to accelerate the training, and the complete retrosynthetic reaction process is replaced by multiple layers of molecular retrosynthetic routes. After the training of one layer of molecular retrosynthesis route is completed, a representative molecule is selected by molecular screening and used as a starting molecule in a next layer of molecular retrosynthetic route, which effectively improves the exploration efficiency of the molecular retrosynthetic route, whereby accurate molecular cost information is more efficiently extracted. The layered approach greatly reduces the computational overhead brought about by determining the molecular retrosynthetic route, and reduces the time for determining the molecular retrosynthetic route while the accuracy of the molecular retrosynthetic route is ensured.

According to an embodiment, an apparatus for determining a molecular retrosynthetic route is also provided. The apparatus includes:

a receiving module, configured to receive molecular expression information of a target molecule, the molecular expression information representing a three-dimensional chemical structure of the target molecule;

an input module, configured to input the molecular expression information of the target molecule into a neural network for determining a molecular retrosynthetic route;

a decomposition path determining module, configured to determine a target decomposition path of the target molecule based on the neural network for determining a molecular retrosynthetic route, the target decomposition path being a decomposition path with a minimum decomposition cost value among at least one decomposition path; and

a route determining module, configured to obtain molecular retrosynthetic route information of the target molecule based on the target decomposition path.

In one embodiment, the neural network for determining a molecular retrosynthetic route includes a first neural network and a second neural network; and the decomposition path determining module is further configured to execute operations of: outputting cost value information of an upper-layer retrosynthetic route of the target molecule based on the first neural network; determining a first decomposition path of the target molecule based on the cost value information of the upper-layer retrosynthetic route of the target molecule; determining molecular expression information of a molecule for which a further decomposition method exists among molecules obtained by decomposing the target molecule based on the first decomposition path; inputting the molecular expression information of the molecule for which a further decomposition method exists into the second neural network; outputting cost value information of a lower-layer retrosynthetic route of the target molecule based on the second neural network; determining a second decomposition path of the target molecule based on the cost value information of the lower-layer retrosynthetic route of the target molecule;

and determining the target decomposition path of the target molecule based on the first decomposition path and the second decomposition path.

The apparatus provided in the foregoing embodiments is described by using division into the foregoing functional modules as an example. In actual applications, the foregoing functions may be allocated to and completed by different functional modules according to requirements, that is, the internal structure of the apparatus is divided into different functional modules, to complete all or some of the foregoing described functions. In addition, the apparatus and the corresponding method embodiments provided in the foregoing embodiments belong to the same concept. For the specific implementation process, reference may be made to the method embodiments, and details are not described herein again.

When the computer device is configured as a server, FIG. 9 is a schematic structural diagram of a server according to embodiment of this application. The server 900 may vary greatly due to different configurations or different performance, and can include one or more central processing units (CPUs) 901 and one or more memories 902. The memory 902 stores at least one computer-readable instruction, the at least one computer-readable instruction being loaded and executed by the one or more processors 901 to implement the training method for a neural network for determining a molecular retrosynthetic route and the method for determining a molecular retrosynthetic route that are provided in the foregoing method embodiments, certainly, the server 900 may also have a wired or wireless network interface, a keyboard, an input/output interface and other components to facilitate input/output. The server 900 may also include other components for implementing device functions. Details are not described herein.

The embodiments of this application further provide one or more computer-readable storage media. The one or more computer-readable storage media are applicable to a computer device. The one or more computer-readable storage media store at least one computer-readable instruction. The at least one computer-readable instruction is loaded and executed by one or more processors to implement the operations executed by a computer device in the methods of the above embodiments.

An embodiment of this application further provides a computer program product, including computer instructions, the computer instructions being stored in a computer-readable storage medium. One or more processors of the computer device reads the computer-readable instructions from the computer-readable storage medium, and the one or more processor execute the computer-readable instructions to cause the computer device to perform the steps in the methods provided in the above optional implementations.

A person of ordinary skill in the art may understand that all or some of the steps of the embodiments may be implemented by hardware or a computer-readable instruction instructing related hardware. The computer-readable instruction may be stored in one or more computer-readable storage media. The storage medium may be an ROM, a magnetic disk, an optical disc, or the like.

The foregoing descriptions are merely examples of the embodiments of this application, but are not intended to limit this application. Any modification, equivalent replacement, or improvement made without departing from the spirit and principle of this application shall fall within the protection scope of this application.

**Claims**

1. A training method for a neural network configured to determine a molecular retrosynthetic route, performed by a computer device, the method comprising:

   determining a first decomposition path of each of a plurality of first molecules based on molecular expression information of each of the plurality of first molecules;
   obtaining a first cost dictionary based on the first decomposition paths of the first molecules, the first cost dictionary comprising the molecular expression information of each of the first molecules and cost value information of each of the first molecules, and the cost value information of each first molecule representing a cost value required to decompose the first molecule according to the first decomposition path of the first molecule;
   determining molecular expression information of at least one second molecule based on the first decomposition paths of the first molecules, each of the second molecules being a molecule that is capable of being decomposed into obtainable molecules, among molecules obtained by decomposing each of the first molecules based on the first decomposition path of each of the first molecules;
   determining a plurality of third molecules from the second molecules, each of the third molecules representing a class of the second molecules;
   obtaining a second cost dictionary based on a second decomposition path of each of the third molecules, the second cost dictionary comprising molecular expression information of each of the third molecules and cost value information of each of the third molecules, and the cost value information of each third molecule representing a cost value required to decompose the third molecule according to the second decomposition path of the third molecule; and

performing training based on the first cost dictionary and the second cost dictionary to obtain a target neural network, the target neural network being configured to output cost value information of a target molecule according to input molecular expression information of the target molecule, the cost value information of the target molecule being used for synthesizing a retrosynthetic route for the target molecule.

2. The method according to claim 1, wherein the determining a first decomposition path of each of a plurality of first molecules based on molecular expression information of the plurality of first molecules comprises:

obtaining a first initial cost value function of each of the first molecules based on the molecular expression information and cost value reference information of each of the first molecules;
for each of the first molecules, in response to completing decomposition of a decomposition level of the first molecule, updating the first initial cost value function of the first molecule to obtain a first target cost value function of the first molecule based on a decomposition cost value corresponding to a decomposition path of the decomposition level of the first molecule, the first target cost value function being used for determining a decomposition path with a minimum decomposition cost value for the first molecule; and
in response to a decomposition task of a first molecule satisfying a target decomposition condition, determining a first decomposition path of the first molecule based on the first target cost value function of the first molecule.

3. The method according to claim 2, wherein the obtaining a first initial cost function of each of the first molecules based on the molecular expression information and cost value reference information of each of the first molecules comprises:

dividing the decomposition task of each of the first molecules into a plurality of first subtasks based on the molecular expression information of the first molecules, the decomposition task being dividing the first molecule according to the decomposition path;
allocating the first subtasks to a plurality of computing nodes, so that the computing nodes calculate and return the first initial cost value functions of the first molecules, the first initial cost value functions being obtained by calculating by the corresponding computing nodes based on molecular cost value reference information, and the molecular cost value reference information representing decomposition accessibility of the molecule; and
receiving the first initial cost value functions returned by the computing nodes.

4. The method according to claim 1, wherein the obtaining a first cost dictionary based on the first decomposition paths of the first molecules comprises:

determining cost value information of each of the first molecules based on the first decomposition path of each of the first molecules; and
obtaining the first cost dictionary according to the molecular expression information of each of the first molecules and the cost value information of each of the first molecules.

5. The method according to claim 1, wherein the determining a plurality of third molecules from the second molecules, each of the third molecules representing a class of the second molecules, comprises:

clustering the second molecules to obtain a plurality of sets, each of the sets comprising at least one second molecule with similar molecular structure; and
obtaining the plurality of third molecules by determining a cluster center of each of the sets as a third molecule corresponding to the set, each of the third molecules being a representative molecule in the set to which the third molecule belongs.

6. The method according to claim 1, wherein the performing training based on the first cost dictionary and the second cost dictionary to obtain a target neural network comprises:

training a second neural network based on the molecular expression information and the cost value information of each molecule in the second cost dictionary;
updating the first cost dictionary based on the second cost dictionary to obtain an updated first cost dictionary;
training the first neural network based on the molecular expression information and the cost value information of each molecule in the updated first cost dictionary; and
combining the trained second neural network and the trained first neural network to obtain the target neural network.

**7.** The method according to claim 6, wherein the training a second neural network based on the molecular expression information and the cost value information of each molecule in the second cost dictionary comprises:

inputting the molecular expression information of each molecule in the second cost dictionary into the second neural network to obtain predicted cost value information of each molecule;
determining a model loss of the second neural network based on the predicted cost value information of each molecule and the cost value information of each molecule in the second cost dictionary; and
adjusting a network parameter in the second neural network according to the model loss of the second neural network.

**8.** A method for determining a molecular retrosynthetic route, performed by a computer device, the method comprising:

receiving molecular expression information of a target molecule, the molecular expression information representing a three-dimensional chemical structure of the target molecule;
inputting the molecular expression information of the target molecule into a neural network, wherein the neural network is for determining a molecular retrosynthetic route;
determining a target decomposition path of the target molecule based on the neural network for determining a molecular retrosynthetic route, the target decomposition path being a decomposition path with a minimum decomposition cost value among at least one decomposition path of the target molecule; and
obtaining molecular retrosynthetic route information of the target molecule based on the target decomposition path.

**9.** The method according to claim 8, wherein the determining a target decomposition path of the target molecule based on the neural network for determining a molecular retrosynthetic route comprises:

the neural network for determining a molecular retrosynthetic route comprising a first neural network and a second neural network,
outputting cost value information of an upper-layer retrosynthetic route of the target molecule based on the first neural network;
determining a first decomposition path of the target molecule based on the cost value information of the upper-layer retrosynthetic route of the target molecule;
determining molecular expression information of a molecule on which a decomposition method is able to be applied, among molecules obtained by decomposing the target molecule based on the first decomposition path;
inputting the molecular expression information of the molecule on which a decomposition method is able to be applied into the second neural network;
outputting cost value information of a lower-layer retrosynthetic route of the target molecule based on the second neural network;
determining a second decomposition path of the target molecule based on the cost value information of the lower-layer retrosynthetic route of the target molecule; and
determining the target decomposition path of the target molecule based on the first decomposition path and the second decomposition path.

**10.** A training apparatus for a neural network for determining a molecular retrosynthetic route, disposed in a computer device, the apparatus comprising:

a first determining module, configured to determine a first decomposition path of each of a plurality of first molecules based on molecular expression information of each of the plurality of first molecules;
a first cost dictionary generation module, configured to obtain a first cost dictionary based on the first decomposition paths of the first molecules, the first cost dictionary comprising the molecular expression information of each of the first molecules and cost value information of each of the first molecules, and the cost value information of each first molecule representing a cost value required to decompose the first molecule according to the first decomposition path of the first molecule;
a second determining module, configured to determine molecular expression information of at least one second molecule based on the first decomposition paths of the first molecules, each of the second molecules being a molecule that is capable of being decomposed into obtainable molecules, among molecules obtained by decomposing each of the first molecules based on the first decomposition path of each of the first molecules;
a third determining module, configured to determine a plurality of third molecules from the second molecules, each of the third molecules representing a class of the second molecules;

a second cost dictionary generation module, configured to obtain a second cost dictionary based on a second decomposition path of each of the third molecules, the second cost dictionary comprising molecular expression information of each of the third molecules and cost value information of each of the third molecules, and the cost value information of each third molecule representing a cost value required to decompose the third molecule according to the second decomposition path of the third molecule; and

a training module, configured to perform training based on the first cost dictionary and the second cost dictionary to obtain a target neural network, the target neural network being configured to output cost value information of a target molecule according to input molecular expression information of the target molecule, the cost value information of the target molecule being used for synthesizing a retrosynthetic route for the target molecule.

11. The apparatus according to claim 10, wherein the first determining module comprises:

an obtaining unit, configured to obtain a first initial cost value function of each of the first molecules based on the molecular expression information and cost value reference information of each of the first molecules;

an updating unit, configured to, for each of the first molecules, in response to completing decomposition of a decomposition level of the first molecule, update the first initial cost value function of the first molecule to obtain a first target cost value function of the first molecule based on a decomposition cost value corresponding to a decomposition path of the decomposition level of the first molecule, the first target cost value function being used for determining a decomposition path with a minimum decomposition cost value for the first molecule; and

a determining unit, configured to, in response to a decomposition task of a first molecule satisfying a target decomposition condition, determine a first decomposition path of the first molecule based on the first target cost value function of the first molecule.

12. The apparatus according to claim 11, wherein the obtaining unit is configured to execute operations of:

dividing the decomposition task of each of the first molecules into a plurality of first subtasks based on the molecular expression information of the first molecules, the decomposition task being dividing the first molecule according to the decomposition path;

allocating the first subtasks to a plurality of computing nodes, so that the computing nodes calculate and return the first initial cost value functions of the first molecules, the first initial cost value functions being obtained by calculating by the corresponding computing nodes based on molecular cost value reference information, and the molecular cost value reference information representing decomposition accessibility of the molecule; and

receiving the first initial cost value functions returned by the computing nodes.

13. The apparatus according to claim 10, wherein the first cost dictionary generation module is configured to execute operations of:

determining cost value information of each of the first molecules based on the first decomposition path of each of the first molecules; and

obtaining the first cost dictionary according to the molecular expression information of each of the first molecules and the cost value information of each of the first molecules.

14. The apparatus according to claim 10, wherein the third determining module is further configured to execute operations of: clustering the second molecules to obtain a plurality of sets, each of the sets comprising at least one second molecule with similar molecular structure; and obtaining the plurality of third molecules by determining a cluster center of each of the sets as a third molecule corresponding to the set, each of the third molecules being a representative molecule in the set to which the third molecule belongs.

15. The apparatus according to claim 10, wherein the training module comprises:

a first training unit, configured to train a second neural network based on the molecular expression information and the cost value information of each molecule in the second cost dictionary;

a second cost dictionary updating unit, configured to update the first cost dictionary based on the second cost dictionary to obtain an updated first cost dictionary;

a second training unit, configured to train the first neural network based on the molecular expression information and the cost value information of each molecule in the updated first cost dictionary; and

a combining unit, configured to combine the trained second neural network and the trained first neural network to obtain the target neural network.

**16.** The apparatus according to claim 15, wherein the first training unit is further configured to execute operations of: inputting the molecular expression information of each molecule in the second cost dictionary into the second neural network to obtain predicted cost value information of each molecule; determining a model loss of the second neural network based on the predicted cost value information of each molecule and the cost value information of each molecule in the second cost dictionary; and adjusting a network parameter in the second neural network according to the model loss of the second neural network.

**17.** An apparatus for determining a molecular retrosynthetic route, disposed in a computer device, the apparatus comprising:

a receiving module, configured to receive molecular expression information of a target molecule, the molecular expression information representing a three-dimensional chemical structure of the target molecule;
an input module, configured to input the molecular expression information of the target molecule into a neural network, wherein the neural network is for determining a molecular retrosynthetic route;
a decomposition path determining module, configured to determine a target decomposition path of the target molecule based on the neural network for determining a molecular retrosynthetic route, the target decomposition path being a decomposition path with a minimum decomposition cost value among at least one decomposition path of the target molecule; and
a route determining module, configured to obtain molecular retrosynthetic route information of the target molecule based on the target decomposition path.

**18.** The apparatus according to claim 17, wherein the neural network for determining a molecular retrosynthetic route comprising a first neural network and a second neural network; and the decomposition path determining module is further configured to execute operations of: outputting cost value information of an upper-layer retrosynthetic route of the target molecule based on the first neural network; determining a first decomposition path of the target molecule based on the cost value information of the upper-layer retrosynthetic route of the target molecule; determining molecular expression information of a molecule on which a decomposition method is able to be applied, among molecules obtained by decomposing the target molecule based on the first decomposition path; inputting the molecular expression information of the molecule on which a decomposition method is able to be applied into the second neural network; outputting cost value information of a lower-layer retrosynthetic route of the target molecule based on the second neural network; determining a second decomposition path of the target molecule based on the cost value information of the lower-layer retrosynthetic route of the target molecule; and determining the target decomposition path of the target molecule based on the first decomposition path and the second decomposition path.

**19.** A computer device, comprising one or more processors and a memory, the memory storing at least one computer-readable instruction, the at least one computer-readable instruction being loaded and executed by the one or more processors to implement the method according to any one of claims 1 to 9.

**20.** One or more computer-readable storage media, storing at least one computer-readable instruction, the at least one computer-readable instruction being used for executing the method according to any one of claims 1 to 9.

FIG. 1

Depth
d=0

Depth
d=1

Depth
d=2

Depth
d=5

201

202

Cluster screening

Starting molecule of lower-layer
molecular retrosynthetic tree

Depth
d=0

Depth
d=1

Depth
d=2

Depth
d=5

203

● Product  ⊘ Intermediate product  ⊗ Basic material  ○ Maximum depth

FIG. 2

The server determines a first decomposition path of each of a plurality of first molecules based on molecular expression information of each of the plurality of first molecules — 301

The server obtains a first cost dictionary based on the first decomposition paths of the first molecules, the first cost dictionary comprising the molecular expression information of each of the first molecules and cost value information of each of the first molecules, and the cost value information of each first molecule representing a cost value required to decompose the first molecule according to the first decomposition path of the first molecule — 302

The server determines molecular expression information of at least one second molecule based on the first decomposition paths of the first molecules, each of the second molecules being a molecule that is capable of being decomposed into obtainable molecules — 303

The server determines a plurality of third molecules from the second molecules, each of the third molecules representing a class of the second molecules — 304

The server obtains a second cost dictionary based on a second decomposition path of each of the third molecules, the second cost dictionary comprising molecular expression information of each of the third molecules and cost value information of each of the third molecules, and the cost value information of each third molecule representing a cost value required to decompose the third molecule according to the second decomposition path of the third molecule — 305

The server performs training based on the first cost dictionary and the second cost dictionary to obtain a target neural network, the target neural network being configured to output cost value information of a target molecule according to input molecular expression information of the target molecule — 306

FIG. 3

The server divides the decomposition task of each of the first molecules into a plurality of first subtasks based on the molecular expression information of each of the first molecules, the decomposition task is dividing the first molecule according to the decomposition path /— 401

↓

The server allocates first subtasks to computing nodes, so computing nodes calculate and return first initial cost value functions of first molecules, first initial cost value functions is obtained by calculating by computing nodes based on molecular cost value reference information /— 402

↓

The server receives the first initial cost value functions returned by the computing nodes /— 403

↓

in response to complete decomposition of a decomposition level of a first molecule, the server updates first initial cost value function of the first molecule to obtain first target cost value function of the first molecule based on decomposition cost value of a decomposition path of the decomposition level of the first molecule, the first target cost value function is for determining a decomposition path with minimum decomposition cost value for the first molecule /— 404

↓

in response to a decomposition task of any one of the first molecules satisfying a target decomposition condition, the server determines a first decomposition path of the first molecule based on the first target cost value function of the first molecule /— 405

↓

The server determines cost value information of each of the first molecules based on the first decomposition path of each of the first molecules /— 406

↓

The server obtains a first cost dictionary according to the molecular expression information of each of the first molecules and the cost value information of each of the first molecules, the first cost dictionary including the molecular expression information of each of the first molecules and the cost value information of each of the first molecules /— 407

↓

The server determines molecular expression information of at least one second molecule based on the first decomposition paths of the first molecules, each of the second molecules is a molecule that can be decomposed into obtainable molecules /— 408

↓

The server clusters the second molecules to obtain a plurality of sets, each of the sets including at least one second molecule with similar molecular structure /— 409

↓

The server determines cluster centers of the sets as a plurality of third molecules, each of the third molecules being a representative molecule in the set to which the third molecule belongs /— 410

↓

The server obtains a second cost dictionary based on second decomposition paths of the third molecules, the second cost dictionary includes the molecular expression information of each of the third molecules and cost value information of each of the third molecules, and the cost value information of each third molecule representing a cost value required to decompose the third molecule according to the corresponding second decomposition path /— 411

↓

The server trains a second neural network based on the molecular expression information and the corresponding cost value information of each molecule in the second cost dictionary /— 412

↓

The server updates the first cost dictionary based on the second cost dictionary to obtain an updated first cost dictionary /— 413

↓

The server trains the first neural network based on the molecular expression information and the cost value information of each molecule in the updated first cost dictionary /— 414

↓

The server combines the trained second neural network and the trained first neural network to obtain the target neural network /— 415

FIG. 4

The server divides the decomposition task of each of the third molecules into a plurality of second subtasks based on the molecular expression information of each of the third molecules, the decomposition task is dividing the third molecule according to the decomposition path — 501

The server allocates the second subtasks to a plurality of computing nodes, so that the computing nodes calculate and return the second initial cost value functions of the third molecules, the second initial cost value functions is obtained by calculating by the corresponding computing nodes based on molecular cost value reference information — 502

The server receives the second initial cost value functions returned by the computing nodes — 503

For each third molecules, in response to completing decomposition of a decomposition level of the third molecule, the server updates the second initial cost value function of the third molecule to obtain a second target cost value function of the third molecule based on a decomposition cost value of a decomposition path of the decomposition level of the third molecule, the second target cost value function is used for determining a decomposition path with a minimum decomposition cost value for the third molecule — 504

In response to a decomposition task of any one of the third molecules satisfying a target decomposition condition, the server determines a second decomposition path of the third molecule based on the second target cost value function of the third molecule — 505

The server determines cost value information of each of the third molecules based on the second decomposition path of each of the third molecules — 506

The server obtains a second cost dictionary according to the molecular expression information of each of the third molecules and the cost value information of each of the third molecules, the second cost dictionary includes the molecular expression information of each of the third molecules and the cost value information of each of the third molecules — 507

FIG. 5

FIG. 6

A server receives molecular expression information of a target molecule, the molecular expression information represents a three-dimensional chemical structure of the target molecule — 701

The server inputs the molecular expression information of the target molecule into a neural network for determining a molecular retrosynthetic route — 702

The server determines a target decomposition path of the target molecule based on the neural network for determining a molecular retrosynthetic route, the target decomposition path is a decomposition path with a minimum decomposition cost value among at least one decomposition path — 703

The server obtains molecular retrosynthetic route information of the target molecule based on the target decomposition path — 704

FIG. 7

Training apparatus for neutral
network for determining molecular
retrosynthetic route

First determining module ⟋ 801

First cost dictionary
generation module ⟋ 802

Second determining
module ⟋ 803

Third determining
module ⟋ 804

Second cost dictionary
generation module ⟋ 805

Training module ⟋ 806

FIG. 8

900

Server

Processor ⟋ 901

Memory ⟋ 902

FIG. 9

## INTERNATIONAL SEARCH REPORT

| International application No. |
|---|
| **PCT/CN2021/122724** |

**A. CLASSIFICATION OF SUBJECT MATTER**

G16C 20/50(2019.01)i

According to International Patent Classification (IPC) or to both national classification and IPC

**B. FIELDS SEARCHED**

Minimum documentation searched (classification system followed by classification symbols)

G16C, G06F, G06N

Documentation searched other than minimum documentation to the extent that such documents are included in the fields searched

Electronic data base consulted during the international search (name of data base and, where practicable, search terms used)

DWPI, CNABS, WPABS, CNTXT, USTXT, CNKI: 分子, 化学, 化合, 合成, 逆合成, 神经网络, 路线, 路径, 拆解, 分解, 字典, 代价, 代价值, 聚类, 训练, 三维, molecular, chemical, synthetic, inverse synthetic, neural network, route, path, disassembly, decomposition, dictionary, cost, value, clustering, training, three dimensional

**C. DOCUMENTS CONSIDERED TO BE RELEVANT**

| Category* | Citation of document, with indication, where appropriate, of the relevant passages | Relevant to claim No. |
|---|---|---|
| PX | CN 112037868 A (TENCENT TECHNOLOGY SHENZHEN CO., LTD.) 04 December 2020 (2020-12-04)<br>　　description paragraphs 23-172 | 1-20 |
| A | CN 111524557 A (TENCENT TECHNOLOGY SHENZHEN CO., LTD.) 11 August 2020 (2020-08-11)<br>　　entire document | 1-20 |
| A | CN 110728047 A (INSTITUTE OF CHEMICAL MATERIALS, CHINA ACADEMY OF ENGINEERING PHYSICS) 24 January 2020 (2020-01-24)<br>　　entire document | 1-20 |
| A | CN 109872780 A (BEIJING DEEP INTELLIGENT PHARMA TECHNOLOGY CO., LTD.) 11 June 2019 (2019-06-11)<br>　　entire document | 1-20 |
| A | WO 2020163860 A1 (GOOGLE LLC.) 13 August 2020 (2020-08-13)<br>　　entire document | 1-20 |

☑ Further documents are listed in the continuation of Box C. ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 January 2022** | **14 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/122724** |

**C.     DOCUMENTS CONSIDERED TO BE RELEVANT**

| Category* | Citation of document, with indication, where appropriate, of the relevant passages | Relevant to claim No. |
| --- | --- | --- |
| A | WO 2020023650 A1 (WUXI NEXTCODE GENOMICS USA INC) 30 January 2020 (2020-01-30)<br>      entire document | 1-20 |

Form PCT/ISA/210 (second sheet) (January 2015)

**INTERNATIONAL SEARCH REPORT**
Information on patent family members

International application No.

**PCT/CN2021/122724**

| Patent document cited in search report | | | Publication date (day/month/year) | Patent family member(s) | | | Publication date (day/month/year) |
|---|---|---|---|---|---|---|---|
| CN | 112037868 | A | 04 December 2020 | CN | 112037868 | B | 12 February 2021 |
| CN | 111524557 | A | 11 August 2020 | None | | | |
| CN | 110728047 | A | 24 January 2020 | None | | | |
| CN | 109872780 | A | 11 June 2019 | None | | | |
| WO | 2020163860 | A1 | 13 August 2020 | EP | 3906559 | A1 | 10 November 2021 |
| | | | | CA | 3129069 | A1 | 13 August 2020 |
| | | | | KR | 20210119479 | A | 05 October 2021 |
| WO | 2020023650 | A1 | 30 January 2020 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 2020112189912